(19) Europäisches Patentamt / European Patent Office / Office européen des brevets

(11) **EP 3 313 374 B1**

(12) **EUROPEAN PATENT SPECIFICATION**

(45) Date of publication and mention of the grant of the patent:
**12.02.2025 Bulletin 2025/07**

(21) Application number: **16817377.1**

(22) Date of filing: **29.06.2016**

(51) International Patent Classification (IPC):
*A61K 9/00* (2006.01)  *A61K 9/50* (2006.01)
*A61K 47/34* (2017.01)  *A61K 9/16* (2006.01)
*A61K 31/536* (2006.01)  *A61K 47/32* (2006.01)
*A61K 47/36* (2006.01)  *A61K 9/107* (2006.01)

(52) Cooperative Patent Classification (CPC):
**A61K 9/1075; A61K 9/167; A61K 9/1676;**
**A61K 31/536; A61K 47/32; A61K 47/36**

(86) International application number:
**PCT/IL2016/050692**

(87) International publication number:
**WO 2017/002114 (05.01.2017 Gazette 2017/01)**

(54) **AMPHIPHILIC POLYMERS ENCAPSULATING THERAPEUTICALLY ACTIVE AGENTS, PROCESS OF PREPARING SAME AND USE THEREOF**

IN AMPHIPHILEN POLYMEREN VERKAPSELTE WIRKSTOFFE, VERFAHREN ZUR HERSTELLUNG DAVON UND VERWENDUNG DAVON

POLYMÈRES AMPHIPHILES ENCAPSULANT DES AGENTS THÉRAPEUTIQUEMENT ACTIFS, LEUR PROCÉDÉ DE PRÉPARATION ET UTILISATION ASSOCIÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.06.2015 US 201562185777 P**

(43) Date of publication of application:
**02.05.2018 Bulletin 2018/18**

(73) Proprietor: **Technion Research & Development Foundation Limited**
**3200004 Haifa (IL)**

(72) Inventors:
• **SOSNIK, Alejandro Dario**
  **3299414 Haifa (IL)**
• **MENAKER RASKIN, Maya**
  **3522221 Haifa (IL)**

(74) Representative: **Boult Wade Tennant LLP**
**Salisbury Square House**
**8 Salisbury Square**
**London EC4Y 8AP (GB)**

(56) References cited:
**US-A1- 2007 253 899**

• **TORCHILIN V.P. ET AL.: "Applications of polymer micelles for imaging and drug delivery", WIRES NANOMED NANOBIOTECHNOL, vol. 7, no. 5, 15 February 2015 (2015-02-15), pages 691 - 707, XP055342733**
• **SOSNIK A. ET AL.: "Polymeric micelles in mucosal drug delivery: Challenges towards clinical translation", BIOTECHNOLOGY ADVANCES, MARINE DRUGS, vol. 33, 15 January 2015 (2015-01-15), pages 1380 - 1392, XP055342734**
• **SZYMANSKA E. ET AL.: "Stability of Chitosan - A Challenge for Pharmaceutical and Biomedical Applications", MAR. DRUGS, vol. 13, 1 April 2015 (2015-04-01), pages 1819 - 1846, XP055342735**

**Description**

FIELD OF INVENTION

This invention is directed to, *inter alia*, amphiphilic polymeric compositions and uses thereof, such as for encapsulating and releasing therapeutically active agents.

BACKGROUND OF THE INVENTION

"Drug release" refers to the process in which drug solutes migrate from its position within the release-modifying compound or compounds into the medium. Drug release is an important topic in the field of drug delivery for decades. With advancement in material design and engineering, novel materials with increasing complexity and functions have been introduced into the development of drug delivery devices and systems.

Molecules, macromolecules and polymers are vastly used to control drug release. Controlling drug release has direct impact on the bioefficacy, the clinical effect and often times on the quality of life of the target patient population. Non-limiting factors that influence drug release include solute diffusion and polymeric matrix swelling.

Nano- and micro- particles hold promise for controlled and targeted drug release and delivery. An ideal drug carrier should not exert harmful effects on normal cells. It should also satisfy requirements of stability, in vivo biocompatibility, and ability of targeted on-demand release.

U.S. Patent No. 5,173,322 teaches the production of reformed casein micelles and the use of such micelles as a complete or partial replacement of fat in food product formulations.

International Publication NO. WO 2003/105607 discloses nano-sized self-assembled structured concentrates and their use as carriers of active materials, particularly lipophilic compounds suitable for pharmaceutical or cosmetic applications or as a food additive.

WO 2001/087227 discloses polymeric micelles which are pH and/or temperature sensitive, and which are used to increase potency of therapeutic agents.

US 2007/253899 discloses functionalized polymeric micelles for imaging or selectively targeting tissue. Specifically, incorporation of a targeting moiety (such as a peptide), or an imaging moiety (such as radioactive isotopes) by either covalent attachment, or by electrostatic binding to the outer shell of the particle is disclosed.

Torchilin V.P. et al., Wires Nanomed Nanobiotechnol., vol. 7, p. 691-707, 2015 discloses core-corona polymeric micelles which serve as a reservoir for drugs, protects the cargo from its environment during circulation, and is responsible for pharmacological activity. Torchilin et al., discloses surface engineering of the polymeric micelles for inducing targeted delivery of the bioactive agents. Specifically, covalent attachment of (i) targeting ligands for enhancing intracellular accumulation at a target site; or of (ii) chelating agents for incorporation of imaging moieties is disclosed.

Sosnik A. et al., Biotechnology Advances, Mrine Drugs, vol. 35, p. 1380-1392, 2015 discloses *inter alia* chitosan copolymer modified with stearic acid residues capable of core-corona micelle formation. Specifically, Sosnik A. et al. discloses covalent corona cross-linking by glutaraldehyde.

Szymanska E. et al., Mar. Drugs, vol. 13, p. 1819-18446, 2015 discloses use of ionic crosslinkers to form a network of ionic bridges between negatively charged components and the positively charged chitosan chains, so as increase long-term stability of chitosan products.

SUMMARY OF THE INVENTION

This invention is directed to, *inter alia,* amphiphilic graft polymeric compositions and uses thereof, such as for encapsulating and releasing therapeutically active agents. According to some embodiments of the present invention, there is provided a composition-of-matter comprising

one or more amphiphilic graft polymers, the amphiphilic graft polymers comprising a hydrophobic domain attached to a side chain group of a hydrophilic domain, wherein:

(a) said hydrophobic domain is selected from the group consisting of a polyester, a polyacrylamide, a polymethacrylate, and a polyacrylate or a combination thereof;
(b) said hydrophilic domain is selected from the group consisting of a polycarboxylic acid, a polyol, a polysaccharide or a combination thereof;

and said one or more amphiphilic graft polymers are in a form of a multi-micellar particle comprising a hydrophobic core and a non-covalently cross-linked hydrophilic corona, wherein the non-covalently cross-linked is via any one of: ionotropic cross-linking, and complexation interactions.

In some embodiments, the core is characterized by a structure having a cavity that ranges from 1 nm to 10 um in diameter, optionally said core being characterized by a structure having a cavity that ranges from 50 nm to 300 nm in diameter.

In some embodiments, the hydrophobic domain comprises a polymer selected from the group consisting of: N-isopropylacrylamide, methyl acrylate, ethyl acrylate, propyl acrylate, butyl acrylate, methyl methacrylate, ethyl methacrylate, propyl methacrylate, butyl methacrylate, acrylic acid, methacrylic acid, quaternary ammonium-modified acrylate, quaternary ammonium modified-methacrylate, acrylamide, caprolactone, lactide, valeronolactone, and optionally wherein the hydrophobic domain is in the range of 10% to 90%, by weight, of the amphiphilic block polymer.

In some embodiments, a hydrophobic polymeric backbone of said hydrophobic domain is attached to a side-chain group of said polymeric backbone of said hydrophilic domain *via*: (a) a bifunctional coupling agent selected from the group consisting of: diisocyanates, disilanes, dialkoxysilanes, diglycidyl ethers; (b) a coupling agent comprising a higher functionality than two; or (c) condensation agent selected from the group consisting of dicyclohexylcarbodiimide, O-(benzotriazol-1-yl)-N,N,N',N'-tetramethyluronium tetrafluoroborate, or a combination thereof.

In some embodiments, the hydrophilic domain comprises one or more monomeric units derived from a polymer selected from the group consisting of: polyvinyl alcohol (PVA), chitosan, alginate, galactomannan, hydroxyproylmethyl cellulose, carboxymethyl cellulose, glucomannan, guar gum, xanthan gum, pectin, hyaluronic acid, chondroitin sulfate, dermatan sulfate.

In some embodiments, the composition-of-matter of the invention is characterized as being adhesive to a mucosal tissue.

In some embodiments, the amphiphilic graft polymers are characterized as being substantially non-biodegradable in a physiologic environment for a period of at least 24 hours.

In some embodiments, the amphiphilic graft polymers are characterized by a hydrophilic-lipophilic balance (HLB) value that ranges from 1 to 24, optionally wherein a plurality of amphiphilic graft polymers is self-assembled so as to form said multi-micellar particle.

In some embodiments, the composition-of-matter of the invention comprises one or more active agents, each of said active agents being independently encapsulated within said core, optionally wherein said one or more active agents are stably encapsulated within said core in a physiological environment for at least 24 h, optionally, wherein said one or more active agents are selected from the group consisting of a pharmaceutically active agent, a labeling agent, a diagnostic agent, a prophylactic agent, a surface-modifying agent, a tumor-targeting- ligand or moiety, optionally, wherein said one or more active agents are selected from the group consisting of a pharmaceutically active agent, a labeling agent, a diagnostic agent, a prophylactic agent, a surface-modifying agent, a tumor-targeting- ligand or moiety, and optionally wherein said one or more agents are water-insoluble agent.

In some embodiments, at least portion of said corona is positively or negatively charged, optionally wherein at least portion of said corona is non-covalently linked to one or more material selected from the group consisting of a cation, an anion, a polycation, a polyanion, a complexation agent.

In some embodiments, the composition-of-matter of the invention is for use as a medicament.

In another aspect, there is provided a pharmaceutical product, comprising the composition-of-matter of claim 9, optionally said product being a pharmaceutically acceptable injectable matrix, optionally said product being formulated for oral or nasal administration.

In another aspect, there is provided a pharmaceutical product, comprising the composition-of-matter of the invention, for use in the treatment of a medical condition treatable by said therapeutically active agent, and optionally for use in monitoring or treating a medical condition, said product being packaged in a packaging material and identified in print, in or on said packaging material.

In another aspect, there is provided a process of preparing an amphiphilic graft polymer comprising the steps of:

grafting a hydrophobic polymeric backbone to a hydrophilic polymeric backbone in a dispersion, thereby forming said amphiphilic graft polymer configured to form a hydrophobic core and a hydrophilic corona;
dispersing the amphiphilic graft polymer in an aqueous medium at a concentration above a predefined minimal concentration;
adding an active agent to the dispersion, thereby encapsulating the active agent within the hydrophobic core; and
adding a non-covalent crosslinker to the dispersion, so as to form a non-covalent cross-link of at least portion of the hydrophilic corona, wherein said non-covalent cross-link is via any one of: ionotropic cross-linking, and complexation interactions;
thereby forming said amphiphilic graft polymer being in form of a closed core-corona and self-assembled structure further encapsulating one or more active agents.

In some embodiments, the predefined concentration is critical micelle concentration (CMC), optionally said method further comprising a step of heating an aqueous solution containing said amphiphilic block polymer to a temperature that ranges from about 30 °C to about 50 °C, prior to the step of adding the active agent to the dispersion, optionally said method further comprising a step of cooling the dispersion to a temperature lower than 30 °C, thereby stabilizing the amphiphilic block polymer, and optionally said method further comprising a step of diluting the dispersion to a final concentration below the predefined concentration, following the step of adding an active agent to the dispersion thereby stabilizing the amphiphilic block polymer; further optionally wherein said closed core-corona and self-assembled structure is the multi-micellar particle of the invention.

Unless otherwise defined, all technical and/or scientific terms used herein have the same meaning as commonly understood by one of ordinary skill in the art to which the invention pertains. In addition, the materials, methods, and examples are illustrative only and are not intended to be necessarily limiting.

BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description together with the drawings makes apparent to those skilled in the art how embodiments of the invention may be practiced.

In the drawings:

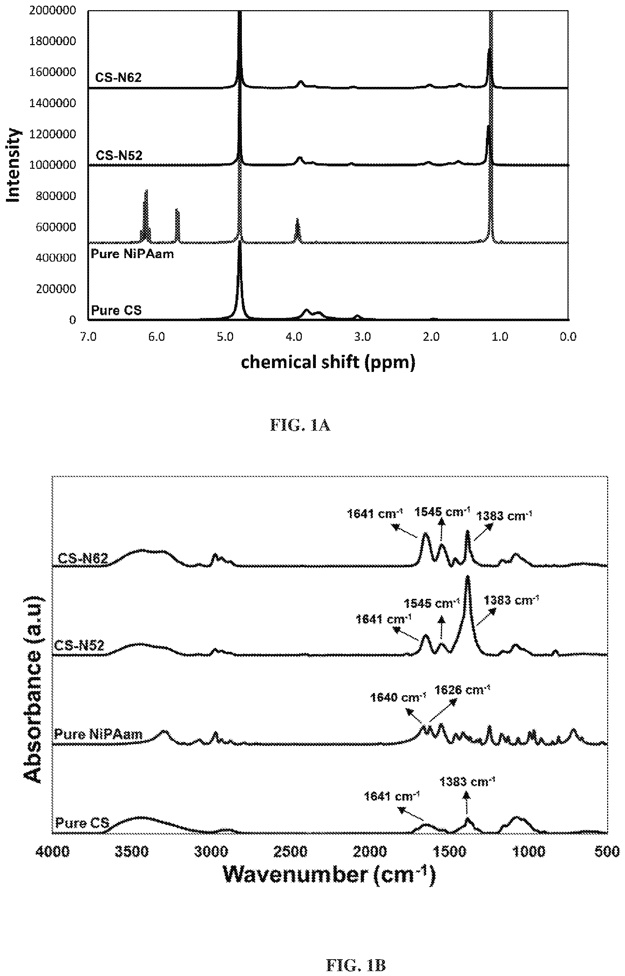

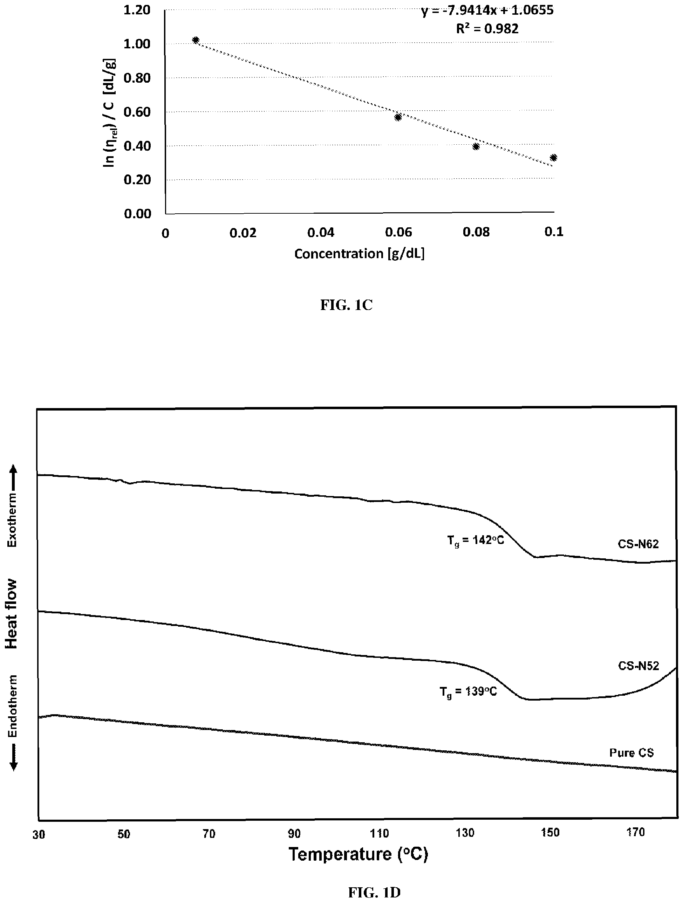

Figures 1A-D present characterizations of the disclosed polymers: $^1$H-NMR spectra of pure CS, pure NiPAam and two CS-g-oligo(NiPAam) copolymers with growing NiPAam content, CS-N52 and CS-N62 (Figure 1A); FTIR spectra of pure CS, pure NiPAam and two CS-g-oligo(NiPAam) copolymers with growing NiPAam content (Figure 1B; FTIR: Fourier Transform Infra-Red); Kraemer plot (ln$\eta$rel/c versus concentration) for CS-N52 copolymer (Figure 1C); Thermal behavior of CS, CS-N52 and CS-N62, as determined by DSC (Figure 1D). "CS" refers to chitosan and "N" refers to N-isopropylacrylamide (NiPAam) and the number (herein 40) refers to percent, by weight, of the N in the polymeric structure.

Figures 2A-B present Nanoparticle Tracking Analysis (NTA) images of non-crosslinked CS-N52 polymeric micelles as described hereinbelow upon formation at 37 °C (Figure 2A) and upon disassembly at 25 °C (Figure 2B).

Figures 3A-B present NTA images of non-crosslinked PVA-N40 polymeric micelles as described hereinbelow upon formation at 37°C (Figure 3A) and upon disassembly at 25 °C (Figure 3B). ("PVA" denotes Poly(vinyl alcohol).

Figures 4A-B present NTA image of non-covalently crosslinked CS-N52 polymeric micelles after incubation at 25 °C for 12 h (Figure 4A) and NTA image of non-crosslinked CS-N52 polymeric micelles upon formation at 37°C (Figure 4B).

Figures 5A-B present NTA image of non-covalently crosslinked PVA-N40 polymeric micelles after incubation at 25 °C for 12 h (Figure 5A) and NTA image of non-crosslinked PVA-N38 polymeric micelles upon formation at 37 °C (Figure 5B) (included for comparison).

Figure 6 presents viability percent of: Caco2 cells after incubation with different non-crosslinked and crosslinked CS-g-oligo(NiPAam) PVA-g-oligo(NiPAam) micelles using the MTT method. An only-medium control was considered 100% viability. "g" denotes grafted.

Figures 7A-C summarizes the percentage of viability of non-crosslinked (Figures 7A, 7B) and crosslinked (Figure 7C) PVA-g-oligo(NiPAam) polymeric micelles with respect to an only-medium control that was considered 100% viability. In addition, the cell viability after exposure to BA (boric acid; the crosslinker) was also assessed (Figure 7B). Non-crosslinked PVA-N56 micelles, non-crosslinked PVA-N68 micelles and crosslinked PVA-N56 micelles that were crosslinked with 0.3% w/v BA. An only-medium control was considered 100% viability.

Figure 8 present viability percent of Caco2 cells after incubation with different non-crosslinked CS-g-PMMA micelles and BA using the MTT method. "PMMA" denotes Poly(methyl methacrylate).

Figures 9A-F presents high-resolution scanning electron microscopy micrographs of pure CS solution (pH 5) (Figure 9A), non-crosslinked CS-N52 (Figures 9B, 9C, and 9E); specimen of never heated copolymer solution (Figure 9B), specimen heated 12 h and then dried 12 h in 37 °C (Figure 9C), specimen heated 12 h in 37 °C and then dried in 25 °C (Figure 9D), specimen prepared after 48 h in 25 °C (Figure 9E), and ionotropically-crosslinked CS-N52 polymeric micelles specimen (Figure 9F). Bars are 2 micrometers.

**Figures 10A-C** present cryo-transmission electron microscopy micrographs of drug-free non-crosslinked CS-N52 micelles (**Figure 10A;** bar is 100 nm) and ionotropically-crosslinked CS-N52 polymeric micelles visualized (**Figures 10B** and **10C;** bars are 100 nm and 200 nm, respectively).

**Figures 11A-C** present high-resolution scanning electron microscopy (HR-SEM) micrographs of non-crosslinked PVA-N66 polymeric micelles. 0.015% w/v (FigureS 11A and 11B) and 0.03% w/v (Figure 11 C). Bar is 200 nm.

**Figures 12A-B** present cryo-transmission electron microscopy micrographs of efavirenz loaded-free CS-N52 micelles as described in the Examples section below: non-crosslinked (**Figure 12A**) and ionotropically-crosslinked (**Figure 12B**). Bar is 200 nm.

**Figure 13** presents *in vitro* drug release profile in phosphate buffer saline (pH 7.4) at 37 °C from 1:50 diluted efavirenz-containing non-crosslinked (♦) and ionotropically-crosslinked (▲) 1% w/v CS-N52 polymeric micelles.

## DETAILED DESCRIPTION OF THE INVENTION

The present invention, in some embodiments thereof, relates to compositions and more specifically, but not exclusively, to compositions for administration of active agents and to uses thereof in the treatment of medical conditions. The present invention, in further embodiments thereof, relates to compositions and their uses for treating medical conditions that are otherwise treatable by parenteral administration of hydrophobic therapeutically active agents and more specifically, but not exclusively, to compositions for oral administration of such therapeutically active agents and to uses thereof in the treatment of medical conditions treatable by these therapeutically active agents.

The present inventors have devised a methodology for successfully stably encapsulating active agents in an amphiphilic polymer comprising a hydrophobic core and a hydrophilic corona. The polymer may have nano-sized or submicron-sized structure. Using this methodology, these polymeric structure encapsulating different drugs have been prepared and characterized, and were further shown to associate to the drug with high affinity thereto.

Before explaining at least one embodiment of the invention in detail, it is to be understood that the invention is not necessarily limited in its application to the details set forth in the following description or exemplified by the Examples. The invention is limited to the scope of the appended claims.

### *Compositions-of-matter*

According to an aspect of some embodiments of the present invention, there is provided a composition-of-matter comprising one or more amphiphilic graft polymers, the amphiphilic polymers comprising a hydrophobic domain and a hydrophilic domain, wherein each of the hydrophobic domain and hydrophilic domain comprises a polymeric backbone; the polymeric backbone of the hydrophobic domain is attached to a side-chain group of the polymeric backbone of the hydrophilic domain; and the amphiphilic graft polymers is configured to form a structure having a hydrophobic core and a hydrophilic corona, wherein at least portion of the hydrophilic corona is non-covalently bonded.

In some embodiments, the hydrophobic domain is configured to form a hydrophobic core and the hydrophilic domain is configured to form the hydrophilic corona.

In some embodiments, the hydrophilic domain is configured to form a hydrophilic core and the hydrophobic domain is configured to form the hydrophobic corona.

In some embodiments, each of the hydrophobic domain and hydrophilic domain comprises at least two monomeric units, and the hydrophobic domain is attached to a side-chain group of the hydrophilic domain.

By "attached to", also referred to herein as "grafted to", it is meant to refer to covalently bound, conjugated, hybridized, or immobilized.

As used herein throughout, the term "polymer" describes an organic substance composed of a plurality of repeating structural units (backbone units) covalently connected to one another.

Hereinthroughout, the term "monomer" refers to a molecule that may bind chemically to other molecules to form an oligomer or a polymer.

The term "monomeric unit" refers to the repeating units, derived from the corresponding monomer. The terms "repeating unit" and "monomeric unit" are used hereinthroughout interchangeably. The polymer comprises the monomeric units. By "derived from" it is meant to refer to the polymeric compound following the polymerization process.

In some embodiments, the polymeric backbone of each of the hydrophobic domain comprises two or more monomeric units.

In some embodiments, the polymeric backbone of each of the hydrophilic domain comprises two or more monomeric units.

The term "amphiphilic polymer" is understood to mean a polymer which comprises at least a hydrophilic part (the term "part" is also referred to hereinthroughout as "block", "domain" or "component", interchangeably) and at least a hydrophobic part. This polymer is water-soluble or water-dispersible, directly or e.g., by means of pre-dissolution in an organic solvent miscible with water.

The polymers disclosed herein can be block polymers which comprise, on the one hand, at least one water-soluble or water-dispersible polymer block and, on the other hand, at least one hydrophobic block.

The term "block copolymer" refers to copolymers wherein monomeric units of a given type are organized in blocks, i.e. monomeric units of the same type are adjacent to each other. To explain further, the term "block copolymer" includes molecules of the type $A_iB_jA_k$, wherein A and B designate distinct types of monomers and the indices i, j, k and 1 are integer numbers having a value of at least 1.

The term "amphiphilic block copolymer" according to some embodiments of the present invention designates block copolymers, comprising or consisting of a hydrophobic part and a hydrophilic part, wherein either or both parts may be made of one or more types of monomeric units, the monomeric units being organized in blocks. For example, the term "amphiphilic block copolymer" may relate to di-block copolymers of the general formula $A_iB_j$, wherein one of $A_i$ or $B_j$ is a hydrophobic polymer and the respective other moiety is a hydrophilic polymer.

The term "amphiphilic block copolymer" according to some embodiments of the present invention designates block copolymers, comprising or consisting of a hydrophobic part (domain) and a hydrophilic part (domain), wherein either or both parts may be made of one or more types of monomeric units, the monomeric units being organized in blocks.

The terms "hydrophilic", "water-soluble", and "water-dispersible" are used hereinthroughout interchangeably.

The polymers employed in the context of the present invention can thus be, in some embodiments, block (or multiblock) polymers comprising, for example, hydrophilic domains alternating with hydrophobic blocks. These polymers can also be provided in the form of grafted polymers, the backbone of which is composed of water-soluble or water-dispersible blocks and carries hydrophobic grafts. In some embodiments, the backbone of the grafted polymers (e.g., water-soluble block) is not covalently crosslinked.

Typically, but not exclusively, the term "hydrophilic domain" may be understood to mean a polymer which, when introduced into water at a concentration equal to 1 %, by weight, results in a macroscopically homogeneous solution. In some embodiments, the transmission of light at a wavelength equal to 500 nm through a sample with a thickness of 1 cm, is at least 10%, which corresponds to an absorbance [abs = -log(transmission)] of e.g., less than 1.5.

Typically, but not exclusively, the term "amphiphilic polymer" may be understood to mean a polymer which, introduced into aqueous solution at 0.05% (by weight), makes it possible to reduce the surface tension of the water at 25°C to a value of less than 50 mN/m, e.g., less than 40 mN/m.

The amount (as active material) of amphiphilic polymer in the composition of the invention can range from e.g., 0.0001% to 30% by weight, with respect to the total weight of the composition.

In some embodiments, the ratio of the amount of hydrophobic domain to the amount of polymer range from 1 to 200 or, in some embodiments, from 1 to 150, by weight.

### *Hydrophilic Domains*

Mention may be made, by way of example and without being limited thereto, of the following water-soluble monomers and their salts which are capable of being employed to form the water-soluble or water-dispersible domains, blocks or units, alone or as mixtures: -(meth)acrylic acid, -(hydroxyethylmeth)acrylate, vinylsulfonic acid and (meth) allylsulfonic acid, vinylphosphonic acid, methylvinylimidazolium chloride, - (meth)acrylamide, vinylpyridine, maleic acid and maleic anhydride, vinyl alcohol of formula $CH_2=CHOH$, N-vinyllactams, such as N-vinylpyrrolidone, N-vinylcaprolactam and N-butyrolactam, water-soluble styrene derivatives, e.g., styrenesulphonate.

In some embodiments, the hydrophilic component of the disclosed amphiphilic graft polymer is a multifunctional polymer.

In some embodiments, the hydrophilic component of the disclosed amphiphilic graft polymer is selected from natural, modified natural, synthetic or semisynthetic polymers.

Non-limiting examples of hydrophilic polymers may be e.g., natural, synthetic or semisynthetic polyols, polycarboxylic acids, polysulfonates, polyamines, polysaccharides, polycyclodextrins, proteins, oligopeptides, polypeptides, oligonucleotides that bears e.g., at least three side-chain reactive functional groups per molecule.

As used herein, functional groups, include, but are not limited to, a hydroxyl group, an amine group, a thiol group, a carboxyl group, a sulfonate group, a double or triple bond group, or any other reactive functional group, or any group having functionality groups higher than one, and combinations thereof.

In exemplary embodiments, the hydrophilic domain comprises monomeric units (or the corresponding polymeric domain derived therefrom) selected from the group consisting of vinyl alcohol, or D-glucosamine (deacetylated unit) and N-acetyl-D-glucosamine (or chitosan).

In some embodiments, the hydrophilic domains may have a molar mass of e.g., between 1000 g/mol and 10,000,000 g/mol when they constitute the water-soluble backbone of the block polymer. In some embodiments, these water-soluble blocks have e.g., a molar mass of between 500 g/mole and 500,000 g/mole when they constitute a block or multiblock polymer.

### Hydrophobic Domains

Typically, but not exclusively, the term "hydrophobic domain" is understood to mean blocks which are soluble or dispersible in fatty substances which are liquid at ambient temperature (e.g., 25°C) or oils, such as alkanes, esters, ethers, triglycerides, silicones or fluorinated compounds or a mixture hydrophobic materials (oils).

The terms "hydrophobic" and "water-insoluble" are used hereinthroughout interchangeably. The term, "water-insoluble" is defined to mean that less than e.g., 5 g, 4 g, 3 g, 2 g, 1 g, 0.5 g, 0.4, g, 0.3 g, 0.2 g, or 0.1 g of the domain is soluble in 100 g of water.

In some embodiments, the hydrophobicity characteristic of the domain is unchanged regardless of temperature (e.g., 20 °C to 40 °C).

In some embodiments, the hydrophobicity characteristic of the domain is maintained at a defined range of temperature (e.g., 20°C to 40°C).

In some embodiments, the hydrophobicity characteristic of the domain is unchanged regardless of the pH (e.g., 5 to 9).

In some embodiments, the hydrophobicity characteristic of the domain is maintained at a defined range of pH (e.g., 5 to 9).

In some embodiments, the hydrophobic domain may comprise one or more segments of a hydrophobic molecule or polymer such as, without limitation, lipids, phospholipids, or any other linear or branched polymer or oligomer.

In exemplary embodiments, the hydrophobic domain comprises or is derived from, without being limited thereto, Poly(aspartic acid), Poly($\beta$-benzyl- $_L$-aspartate), Poly(epsilon-caprolactone) (PCL), Poly(D,L-lactide), Poly(propylene oxide) (PPO), and Poly(methyl methacrylate), or any derivative or block polymer thereof (e.g., Poly(ethylene oxide) (PEO) and poly(propylene oxide) (PPO) block co-polymers).

In exemplary embodiments, the hydrophobic domain is derived from N-isopropylacrylamide (NIPAam) and/or methyl methacrylate (MMA).

In some embodiments, the polymer is thermo-responsive polymer characterized by a defined critical solution temperature (LCST). For example, copolymers of NIPAam and acrylamide (AAm) exhibit a lower critical solution temperature (LCST) that is slightly above body temperature. Below the LCST the polymer undergoes hydration and thus it is water-soluble, whereas above the LCST hydrophobic interactions begin to appear, followed by de-hydration and shrinkage. The nanoshells serve as heat transfer agents within the polymer matrix.

In some embodiments, the hydrophobic domain is a block with at least two repeating units of any polyester, polyether, polycarbonate, polyanhydride, polyamide, polyacrylate, polymethacrylate, or any other hydrophobic homopolymer or heteropolymer, or a mixture thereof or any hydrophobic molecule of a group of fatty acids, fatty alcohols, or any other lipid molecule with at least two carbons in the backbone that is grafted to the side-chain of the hydrophilic multifunctional polymer through the reactive functional groups.

In exemplary embodiments, the hydrophobic domain is derived from NIPAam.

In some embodiments, the molar mass of these hydrophobic blocks is between 100 g/mol and 10,000 g/mol. In some embodiments, the molar mass is between 200 g/mol and 5,000 g/mol.

### Amphiphilic Polymers

In some embodiments, the hydrophobic component of the materials of this invention is directly graft-polymerized to the hydrophilic polymer component by means of any graft polymerization reaction known in the art.

Non-limiting examples of such condensation reaction are selected from coordination polymerization, ring-opening polymerization, free radical polymerization, living polymerization and any other methodology of graft polymerization.

By "living polymerization" it is meant to refer to a form of chain growth polymerization where the ability of a growing polymer chain to terminate has been removed. Living radical polymerization is a type of living polymerization where the active polymer chain end is a free radical.

Living polymerization may be selected from living cationic polymerization, living anionic polymerization, and atom-transfer radical (ATR) polymerization.

Several methodologies of living radical polymerization are known in the art and are conceivable to be applied in the context of the present invention, including, without limitation, reversible-deactivation polymerization, catalytic chain transfer, cobalt mediated radical polymerization, iniferter polymerization, stable free radical mediated polymerization, ATR, reversible addition fragmentation chain transfer (RAFT) polymerization, iodine-transfer polymerization (ITP), selenium-centered radical-mediated polymerization, telluride-mediated polymerization (TERP), and stibine-mediated polymerization.

The term "condensation reaction", also referred to in the art as "step-growth process", and the like, means reaction to form a covalent bond between organic functional groups possessing a complementary reactivity relationship, e.g., electrophile-nucleophile. Typically, the process may occur by the elimination of a small molecule such as water or an alcohol. Additional information can be found in G. Odian, Principles of Polymerization, 3rd edition, 1991, John Wiley & Sons: New York, p. 108.

In some embodiments, the hydrophobic component is graft-polymerized directly to the hydrophilic polymer component without a coupling agent.

In some embodiments, the hydrophobic component is graft-polymerized to the hydrophilic polymer component by a coupling agent.

In some embodiments, the coupling agent is a bifunctional coupling agent.

The term "coupling agent", as used herein, refers to a reagent that can catalyze or form a bond between two or more functional groups intra-molecularly, inter-molecularly or both. Coupling agents are widely used to increase polymeric networks and promote crosslinking between polymeric chains, hence, in the context of some embodiments of the present invention, the coupling agent is such that can promote crosslinking between polymeric chains or between domains within a polymeric structure, or between other chemically compatible functional groups of polymeric chains.

In some embodiments of the present invention, the term "coupling agent" is referred to as "crosslinking agent". In some embodiments, one of the domains serves as the coupling agent or as a crosslinking polymer.

Non-limiting examples of coupling agents include diisocyanates, disilanes, dialkoxysilanes, diglycidyl ethers, or any other bifunctional coupling agent or any other coupling agent with a higher functionality than two or any condensation agent such as dicyclohexylcarbodiimide, O-(benzotriazol-1-yl)-N,N,N',N'-tetramethyluronium tetrafluoroborate, and combinations thereof.

As noted hereinabove, in some embodiments, the hydrophobic domain is grafted to a side chain of the hydrophilic domain.

As noted hereinabove, the hydrophilic domain may form a corona structure, wherein in some embodiments, the hydrophilic corona is non-covalently bonded. As further noted hereinabove, the hydrophobic domain may form a core structure.

In some embodiments, the hydrophobic domain is in the range of 10% to 90%, by weight, of the amphiphilic graft polymer.

In some embodiments, the amphiphilic graft polymer is characterized by a hydrophilic-lipophilic balance (HLB) value that ranges from 1 to 24.

In some embodiments, the amphiphilic graft polymers are characterized by a HLB value that ranges from 4 to 15.

As in all associative chemical reactions, the formation of a bond occurs between two groups within compounds or compositions depending on sufficient proximity therebetween. In the context of some embodiments of the present embodiments, the degree of sufficient proximity depends on the attractive forces that can be exerted by the associating groups and the relative reactivity thereof.

The phrase "attractive force", as used herein, refers to physical forces that span and have an effect over a distance, or field, such as electric and magnetic fields. Associating groups which can exert an attractive force field may attract each other over a definable distance, such as in the case of atoms having electrostatic charges.

The term "proximity" as used herein therefore describes any distance that allows interaction between such associating groups, whereby this distance can be practically null and depends on the presence, type and extent of the attractive forces which can be exerted by and affect the associating groups .

A pair of associating groups on two monomeric units or domains should also be oriented appropriately so as to allow a constructive encounter therebetween which results in the formation of a chemical bond. This is particularly important in cases where the associating groups are characterized by radial asymmetry, directivity, polarity, dipole, vectorial force, effective angle and/or other directional and spatial characteristics. An appropriate orientation is determined by steric constrains, surface accessibility and other structural complementarity considerations as described hereinabove. The term "orientation" therefore refers to a steric location and directionality of an object with reference to another object (herein the associating groups).

Regardless if the associating groups exert an attractive force field which extends beyond the physical boundary of the monomeric units or domains, or the degree of mutual reactivity of the associating groups, the monomeric units or domains must be subjected to suitable conditions which will allow them to associate therebetween. By suitable conditions it is meant that the monomeric units or domains need to be present at an adequate density (concentration) and possess suitable kinetic energy (temperature) so as to produce a sufficient number of events in which the monomeric units or domains come in contact in the chemical sense, interact and associate (joined together). By "interact" it is meant that one or more monomeric units or domains, each having associating groups thereon, while being subjected to suitable conditions as discussed hereinbelow, can come close enough to one another, and at a certain angle range, so as to allow the associating groups to be attached to one another and/or to self-assemble.

In addition to an adequate concentration and suitable temperature, the condition which allows the self-assembly of a chemical structure includes other factors which affect the chemical environment in which the monomeric units or domains are placed. These factors include the type of medium (solvent), the ionic strength and pH of the medium (solutes and buffers) and the presence of other chemical agents such as catalysts, oxidation and reduction agents, and other factors which may affect the reactivity of the associating groups.

The terms "self-assembled", or "self-aggregated", refer to a resulted structure of a self-assembly process based on a series of associative chemical reactions between at least two chemical domains or polymers, which occurs when the associating groups on one chemical domains or polymers are in sufficient proximity and are oriented so as to allow constructive association with another domain or polymer. In other words, an associative interaction means an encounter that results in the attachment of the domains or the polymers to one another.

Closed, hollow and self-assembled chemical structures as described hereinbelow (also referred to as "self-assembled core-corona structures") may be used in a myriad of applications, owing to several of the following most consistent and unique characteristics, such as:

- a capacity to assemble and optionally disassemble under particular chemical and physical conditions;
- a hollow and closed interior;
- a uniform and reproducible distribution of shape, size and composition;
- a defined (e.g., spherical) overall shape; and
- a wide range of controllable sizes.

One of the most intuitive uses of a closed and hollow molecular core that can reversibly self-assemble is a vehicle for substance retention, and subsequent release thereof in or to a chemical, biological or physiological system. Other uses of the closed and hollow structures described herein may utilize the unique structural features of the disclosed amphiphilic graft polymer delineated hereinthroughout. Further embodiments of this aspect are detailed hereinbelow under "Pharmaceutical Composition".

As mentioned hereinabove, in some embodiments, the composition-of-matter is in form of a closed, e.g., hollow, and self-assembled structure, the structure having a hydrophobic core and a hydrophilic corona, wherein the hydrophilic corona is layered in a non-covalent manner. As abovementioned, in some embodiments, the term "layer", or any grammatical derivative thereof, refers to a non-covalent crosslinking structure. In some embodiments, the term "layer", or any grammatical derivative thereof, refers to covalent crosslinking structure.

As used herein, the term "crosslinking structure" includes any molecule, atom, or ion that is capable of forming one or more crosslinks between molecules of the crosslinkable polymer, polymeric domains and/or between two or more atoms in a single molecule of the crosslinkable polymer. The term "crosslinking" is used herein to refer to chemically joining two or more polymer molecules by crosslinks.

In some embodiments, the amphiphilic graft polymer described herein or the composition-of-matter comprising the same are in form solid particles.

In some embodiments, a plurality of amphiphilic graft polymers described herein forms a micelle, a micelle-like or a core-corona structure.

As used herein, the term "micelle" describes a colloidal particle, in a simple arrangement or geometric form, typically spherical, of a specific number of amphiphilic molecules, which forms at a well-defined concentration, called the critical micelle concentration (CMC). The micelle can be a single particle or can form a cluster of several micelles, which interact with one another so as to form a particle having a larger dimension (referred to as "multi-micellar particle").

The phrase "critical micelle concentration" (CMC) describes the concentration of the disclosed amphiphilic block copolymers above which the disclosed amphiphilic block copolymers are present substantially in a micellar form under a given set of conditions. At the vicinity of CMC, sharp change in many experimental parameters may be observed, and this may be measured by a number of techniques that include, but not limited to, surface tension measurements, fluorescence, light scattering, conductivity, osmotic pressure, and the like CMC varies as a function of a number of physical factors such as pH, temperature, ionic strength and pressure.

In some embodiments, the disclosed amphiphilic block copolymer or a plurality thereof form a closed, e.g., hollow, and self-assembled structure e.g., micelle or a micelle-like structure. That is, each self-assembled structure comprises at least e.g., 1, 2, 3, 4, 5, 6, 7, 8, 9, 10, 11, 12, 13, 15, 16, 17, 18, 19, 20, 30 ,40, 50, 60, 70, 80, 90, 100, 1000, 10,000, 100,000 amphiphilic block copolymers.

As used herein, the terms "corona", or "shell", which are used hereinthroughout interchangeably, refer to the sphere (typically the hydrophilic domain(s)) surrounding the core. The term "sphere" is used only for the purpose of illustration and it is to be construed that is not only limited to spherical shape but also includes any shape which may find suitability to at least some embodiments of the present invention.

The term "core" refers to the central region (typically the hydrophobic domain(s)) of the structure, which typically contains the hollow.

The term "closed" as used herein, is a relative term with respect to the size, the shape and the composition of two entities, namely an entity that defines an enclosure (the enclosing entity) and the entity that is being at least partially enclosed therein. In general, the term "closed" refers to a morphological state of an object which has discrete inner and outer surfaces which are substantially disconnected, wherein the inner surface constitutes the boundary of the enclosed area or space. The enclosed area or space may be secluded from the exterior area of space which is bounded only by the outer surface.

In the context of the present invention, the closure of the enclosing entity depends of the size, shape and chemical composition of the entity that is being enclosed therein, such that the enclosing entity may be "closed" for one entity and at the same time be "open" for another entity. For example, structures presented herein are closed with respect to certain chemical entities which cannot pass through their enclosing shell or corona, while the same "closed" structures are not closed with respect to other entities.

For example, the structures of the present embodiments may be closed with respect to, for example, a drug molecule, but non-closed with respect to, for example, a single atom ion or an atom of a noble gas. In the context of the present invention, the same "closed" structures are affected by certain conditions e.g., pH, temperature, concentration, etc.

The terms "hollow" or "hollow sphere" is used only for the purpose of illustration and it is to be construed that is not only limited to spherical shape but also includes any shape which may find suitability to at least some embodiments of the present invention., the same "closed" structures are affected by certain conditions e.g., pH, temperature, concentration, etc.

The term "hollow", as used herein, refers to an object having a vacuous cavity, a gap, a void space or an empty space enclosed within. The term "hollow" is not only limited to spherical shape but also includes any shape which may find suitability to at least some embodiments of the present invention. By "void space" herein it is meant to refer to a polymer-free space or a central cavity. The term hollow is used herein as an illustration and it is to be construed that the core-corona structure are not fully hollow in the core.

In some embodiments, the core is of e.g., spherical, cylindrical, rod, lamellae, irregular or any other morphology.

In some embodiments, the core-corona structure (e.g., micelle) of the disclosed composition remains stable above specific pH condition and/or specific concentration (e.g., CMC).

Typically, but not exclusively, the CMC has a value of e.g., $1 \times 10^{-5}$ % w/v to $1 \times 10^{-2}$ % w/v.

In some embodiments, the core-corona structure (e.g., micelle) of the one or more amphiphilic block copolymers remains stable within a specific range of temperature.

In some embodiments, the non-covalently crosslinking as described hereinabove allows the core-corona structure (e.g., micelle) to remain stable at a concentration lower than the specific concentration. The stability of the core-corona structure may be monitored by methods known in the art e.g., dynamic light scattering (DLS), Nanoparticle Tracking Analysis (NTA), and electron microscopy.

In some embodiments, the hydrophobicity characteristic of the domain (e.g. a hydrophobic domain comprising methyl methacrylate monomeric units) is substantially not temperature- and/or pH- dependent.

In some embodiments, once solubilized in water or in any other aqueous medium, and at a final concentration above a certain concentration and/or at certain range of temperature, the amphiphilic graft polymer(s) of the disclosed composition undergo self-aggregation to form nanoscopic, submicroscopic or microscopic structures.

As used hereinthroughout, the term "stable", or any grammatical derivative thereof, may refer to chemical stability. "Chemical stability" means that an acceptable percentage of degradation of the self-assembled structure disclosed hereinthroughout produced by chemical pathways such as oxidation or hydrolysis is formed. In particular, the self-assembled structure is considered chemically stable if no more than about 10% breakdown products are formed after e.g., two weeks of storage at the intended storage temperature of the product (e.g., room temperature, i.e. 15 °C to 30 °C).

The term "stable", or any grammatical derivative thereof, may also refer to physical stability. The term "physical stability" means that with respect to the self-assembled structure disclosed hereinthroughout, an acceptable percentage of aggregates (e.g., dimers, trimers and larger forms) remains formed. In particular, a formulation is considered physically stable if at least about 15% of the aggregates remain formed after e.g., two weeks of storage at the intended storage temperature of the product (e.g., room temperature).

The term "stable" may also refer to the active or therapeutic agent (as described hereinbelow) encapsulated within the self-assembled structure, meaning that at least about 65% of therapeutic agent remains chemically and physically stable after e.g., one month of storage at room temperature.

In some embodiments, at least 70 % of the self-assembled core-corona structures are characterized by a hydrodynamic size (also referred to hereinthroughout as "hydrodynamic diameter" or, for simplicity, "size" or "diameter") that ranges from 10 nm to 10 μm. In some embodiments, at least 80 % of the particles are characterized by a hydrodynamic size that ranges from 10 nm to 10 μm. In some embodiments, at least 90 % of the self-assembled core-corona structures are characterized by a hydrodynamic size that ranges from 10 nm to 10 μm.

In some embodiments, at least 80 % of the self-assembled core-corona structures are characterized by a hydrodynamic size that ranges e.g., from 10 nm to 10 $\mu$m, or from 10 nm to 1 $\mu$m, or from 20 nm to 500 nm, or from 50 nm to 100 nm, or from 10 nm to 50 nm, or from 100 nm to 1 $\mu$m. In exemplary embodiments, the hydrodynamic size ranges from about 300 nm to about 500 nm.

In some embodiments, a plurality of self-assembled core-corona structures as disclosed herein is characterized by a narrow hydrodynamic size distribution.

As used herein "narrow hydrodynamic size distribution" is characterized by e.g., at least 60 %, at least 70 %, at least 80 %, at least 90 %, of the particles having a hydrodynamic size that varies within a range of less than 25 %.

In some embodiments, the "narrow hydrodynamic size distribution" is characterized by size distribution of at least 80% of the particles varying within a range of less than e.g., 60 %, 50 %, 40 %, 30 %, 20 %, or 10 %, including any value therebetween.

In some embodiments, the "narrow hydrodynamic size distribution" is characterized by size distribution of at least 80 % of the self-assembled core-corona structures varying within a range of less than e.g., 60 %, 50 %, 40 %, 30 %, 20 % or 10 %.

As described hereinabove, in some embodiments of the present invention, the hydrophilic corona is configured to crosslink a layer or a substance in non-covalent manner.

By "non-covalent manner" it is meant to refer to binding, by any non-covalent interaction, to another molecule, ion, complex or substance.

The non-covalent interactions include, but are not limited to, ionotropic interaction, complexation interaction, electrostatic interactions, hydrogen bonds, receptor-substrate interactions, or any other non-covalent crosslinking and combinations thereof. For example, saccharide unit(s), such as chitosan, may comprise cationic groups binding to anions.

The term "ion", or any grammatical derivative thereof, is used in its conventional sense to refer to a charged atom or molecule, i.e., an atom or molecule (organic or inorganic) that contains an unequal number of protons and electrons. Positive ions contain more protons than electrons, and negative ions contain more electrons than protons. An ion of the present invention may be singly charged, or it may have a multiple charge i.e., may be monovalent, divalent, trivalent or quadrivalent.

In some embodiments, at least e.g., 10 %, 20 %, 30 %, 40 %, 50 %, 60 %, 70 %, 80 %, 90 %, or 95 % of a surface of the corona is non-covalently crosslinked.

In some embodiments, the amphiphilic graft polymer or the composition-of-matter comprising thereof is biodegradable. In some embodiments, the amphiphilic graft polymer or the composition-of-matter comprising thereof is not biodegradable. In this context, the "amphiphilic graft polymer or the composition-of-matter comprising thereof" may refer to hydrophilic component of the composition-of-matter and/or the hydrophobic component.

As used herein, the term "biodegradable" describes a substance which can decompose under physiological and/or environmental conditions into breakdown products. Such physiological and/or environmental conditions include, for example, hydrolysis (decomposition *via* hydrolytic cleavage), enzymatic catalysis (enzymatic degradation), and mechanical interactions. Typically, but not exclusively, this term refers to substances that decompose under these conditions such that e.g., 50 weight percent of the substance decompose within a time period shorter than one year.

The term "biodegradable" as used in the context of embodiments of the invention, also encompasses the term "bioresorbable", which describes a substance that decomposes under physiological conditions to break down products that undergo bioresorption into the host-organism, namely, become metabolites of the biochemical systems of the host-organism.

In some embodiments, at least e.g., 50 %, 60 %, 70 %, 80 %, 90 % or 99 % of plurality of the disclosed amphiphilic graft polymers is characterized by a low dispersity index (Đ). Herein, the "disclosed amphiphilic graft polymers" may refer to the amphiphilic graft polymers prior to their non-covalently bonding, or, in some embodiments, following the non-covalently bonding as described hereinthroughout.

As used herein, "dispersity index", also termed in the art: "polydispersity index" (denoted hereinthroughout as: "Đ" or "PDI") refers to a measure of the distribution of molecular mass in a given polymer sample. The dispersity index is calculated by dividing the weight average molecular weight (Mw) by the number average molecular weight (Mn). As used herein, the term "weight average molecular weight" generally refers to a molecular weight measurement that depends on the contributions of polymer molecules according to their sizes. As used herein, the term "number average molecular weight" generally refers to a molecular weight measurement that is calculated by dividing the total weight of all the polymer molecules in a sample with the total number of polymer molecules in the sample. These terms are known by those of ordinary skill in the art.

Đ has a value always greater than 1, but as the polymer chains approach uniform chain length, the value of D approaches unity (1). Homogenous size distribution of the amphiphilic graft polymers may contribute, *inter alia,* to a more defined biodistribution.

As used herein "low Đ value" refers to a value below 3. For example a "low Đ value" may be 2.99, 2.98, 2.97, 2.96, 2.95, 2.94, 2.93, 2.92, 2.91, 2.9, 2.8, 2.7, 2.6, 2.5, 2.4, 2.3, 2.2, 2.1, 2, 1.9, 1.8, 1.7, 1.6, 1.5, 1.4, 1.3, 1.2, 1.19, 1.18, 1.17, 1.16, 1.15, 1.14, 1.13, 1.12, 1.11, 1.1, 1.09, 1.08, 1.07, 1.06, 1.05, 1.04, 1.03, 1.02, 1.01, including any value therebetween.

In dynamic light scattering (DLS), the (absolute) width of the distribution can be compared to the mean, and a relative DLS-PDI = width/mean is obtained. For a theoretical Gaussian distribution the overall polydispersity is the relative polydispersity of the distribution. Traditionally, this overall polydispersity has also been converted into an overall DLS-PDI which is the square of the light scattering polydispersity. For a perfectly uniform sample, the PDI value in the context of DLS would be 0. As demonstrated in the Examples section that follows, in exemplary embodiments, the DLS-PDI has values in the range of e.g., about 0.01 to about 0.5, about 0.02 to about 0.2, or about 0.05 to about 0.1

## Pharmaceutical compositions

According to another aspect of the present invention, there is provided a composition and method of preparing a composition which comprises a core-corona e.g., self-assembled, chemical structure of the amphiphilic block copolymer(s) described hereinabove, and an active agent or otherwise a substance being encapsulated in the chemical structure e.g., within the core.

In some embodiments, the composition comprises about e.g., 0.5%, 1%, 1.5%, 2%, 3%, 3.5%, 4%, 4.5%, 5%, 5.5%, 6%, 6.5%, 7%, 7.5%, 8%, 8.5%, 9%, 9.5%, 10%, 10.5%, 11%, 11.5%, 12%, 12.5%, 13%, 13.5%, 14%, 14.5%, or 15%, of active agent, including any value therebetween, by total dry weight of the composition.

As used herein the terms "pharmaceutical composition" or "pharmaceutical product", which are used hereinthroughout interchangeably, refers to a preparation of one or more of the compositions described herein, or physiologically acceptable salts or prodrugs thereof, with other chemical components including but not limited to physiologically suitable carriers, excipients, lubricants, buffering agents, antibacterial agents, bulking agents (e.g. mannitol), antioxidants (e.g., ascorbic acid or sodium bisulfite), anti-inflammatory agents, anti-viral agents (e.g., efavirenz, darunavir), chemotherapeutic agents (e.g., dasatinib, imatinib, camptothecins), anti-bacterial agents (e.g., rifampicin, bedaquiline), antihistamines and the like. The purpose of a pharmaceutical composition is to facilitate administration of an active compound to a subject. The terms "active compound", "active ingredient", "a therapeutically active agent", "active agent", "biologically active agent", "bioactive agent", and the like are used hereinthroughout interchangeably and refer to a compound, which is accountable for biological effect.

The terms "pharmaceutical composition" or "pharmaceutical product" are also to be construe to encompass a cosmetic or cosmeceutical product and a nutrient or a nutraceutical product.

By "biological effect" it is meant to refer to biological, therapeutic or physiological activity in an organism e.g., following administration thereof to a subject.

The terms "physiologically acceptable carrier" and "pharmaceutically acceptable carrier", which may be interchangeably used, refer to a carrier or a diluent that does not cause significant irritation to an organism and does not abrogate the biological activity and properties of the administered compound.

Herein the term "excipient" refers to an inert substance added to a pharmaceutical composition to further facilitate administration of a drug. Examples, without limitation, of excipients include calcium carbonate, calcium phosphate, various sugars and types of starch, cellulose derivatives, gelatin, vegetable oils and polyethylene glycols.

Techniques for formulation and administration of drugs may be found in "Remington's Pharmaceutical Sciences" Mack Publishing Co., Easton, PA, latest edition, which is incorporated herein by reference.

Pharmaceutical products for use in accordance with the present invention thus may be formulated in a conventional manner using one or more pharmaceutically acceptable carriers comprising excipients and auxiliaries, which facilitate processing of the compounds into preparations which can be used pharmaceutically. Proper formulation is dependent upon the route of administration chosen. The dosage may vary depending upon the dosage form employed, the route of administration utilized and the patient subpopulation (e.g., adult, pediatric, geriatric). The exact formulation, route of administration and dosage can be chosen by the individual physician in view of the patient's condition (see e.g., Fingl et al, 1975, in "The Pharmacological Basis of Therapeutics", Ch. 1 p. 1).

The pharmaceutical composition may be formulated for administration in either one or more of routes depending on whether local or systemic treatment or administration is of choice, and on the area to be treated. Administration may be done orally, by inhalation, or parenterally, for example by intravenous drip or intraperitoneal, subcutaneous, intramuscular or intravenous injection, or topically (including ophthalmically, vaginally, rectally, intranasally). Pharmaceutical composition for intravenous or injectable matrix may comprise an effective amount of a biocompatible, biodegradable controlled release material, the material contained in the polymer is selected from: polyanhydrides, lactic acid and glycolic acid copolymers, polyesters (e.g., polylactic acid, and polyglycolic acid), polyorthoesters, polyols, proteins, polysaccharides, and any mixture thereof.

Formulations for topical administration may include but are not limited to lotions, ointments, gels, creams, suppositories, drops, liquids, sprays and powders. Conventional pharmaceutical carriers, aqueous, powder or oily bases, thickeners and the like may be necessary or desirable.

Compositions or products for oral administration include powders or granules, suspensions or solutions in water or non-aqueous media, sachets, pills, caplets, capsules or tablets. Thickeners, diluents, flavorings, dispersing aids, emulsifiers or binders may be desirable.

Formulations for parenteral administration may include, but are not limited to, sterile solutions or dispersions which may also contain buffers, diluents and other suitable additives. Slow release compositions are envisaged for treatment.

The amount of a composition to be administered will, of course, be dependent on the subject being treated, in the medical condition being treated for, the severity of the affliction, the manner of administration, the judgment of the prescribing physician, etc.

The pharmaceutical product may further comprise additional pharmaceutically active or inactive agents such as, but not limited to, an anti-bacterial agent, an antioxidant, a buffering agent, a bulking agent, a surfactant, an anti-inflammatory agent, an anti-viral agent, a chemotherapeutic agent and an antihistamine.

According to an embodiment of the present invention, the pharmaceutical product described hereinabove is packaged in a packaging material and identified in print, in or on the packaging material, for use in the treatment of a disease or disorder, as described herein.

According to another embodiment of the present invention, the pharmaceutical composition is packaged in a packaging material and identified in print, in or on the packaging material, for use in monitoring a disease or disorder, as described herein.

Products of the present invention may, if desired, be presented in a pack or dispenser device, such as an U.S. Food and Drug Administration (FDA) approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accommodated by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the FDA for prescription drugs or of an approved product insert.

As used herein throughout, the terms "encapsulate" and/or "entrap" and their grammatical derivatives and conjugations, as used in the context of the present embodiments, relate to any form of accommodating a substance, herein the active agent, within a closed e.g., structure, herein the self-assembled structure. In some embodiments, entrapment of the active agent in the self-assembled structure, as in the context of the present embodiments, describes complete integration of the active agent within the structure, such that the entrapped active agents are fully isolated from the surrounding environment as long as the structure is assembled (closed).

As used herein, the terms "encapsulate" and/or "entrap" are meant to encompass cases where the encapsulated entity is solvated, e.g., the encapsulation includes solvent molecules. In cases where the encapsulated entity is surrounded by surface active agents, the encapsulation also includes the surrounding surface active agents.

The encapsulation, according to the present embodiments, is also meant to include the encapsulation of the solvent in which the encapsulation process takes place and/or the various solutes which are present in the solvent in addition to e.g., the chemical monomers and the active agent.

In cases where the active agent is not soluble under the conditions of the self-assembly process, the active agent can be solubilized by means of surface active molecules that surround the molecules of the active agent, which are encapsulated therewith in the encapsulation process.

As described hereinabove, the void within a self-assembled core-corona structure wherein the active agent is encapsulated is set by the size of the core, the type of associating groups, and associating mode therebetween. Hence, the size of the void within the self-assembled structure may be controlled by selecting suitable chemical monomeric units having particular associating groups.

The type of active agent which is suitable for encapsulation within the structure according to the present embodiments depends on several characteristics thereof, such as its size, its solubility in the media in which the self-assembled core-corona structure is formed as well as other chemical compatibility criteria.

In some embodiments, the phrase "active agent", is referred to a "drug", that is a compound which exhibits a beneficial pharmacological effect when administered to a subject and hence can be used in the treatment of a condition that benefits from this pharmacological effect.

That is, in some embodiments, the biologically or therapeutically active agent is selected from the group consisting of: prophylactic drugs, anticancer drug or combination of drugs, vitamins or metabolites thereof (e.g., retinoic acid), monoclonal antibody, SiRNA, RNA, microRNA, DNA, genes, a vaccine, a plasmid, a labeling agent, a diagnostic agent, proteins e.g., enzymes, antigens, a bisphosphonate, an antibacterial, an anti-viral or an antifungal reagent.

For example, a composition which comprises a therapeutically active agent (e.g., a drug) attached to or encapsulated in a self-assembled core-corona structure as described herein, can be efficiently utilized for treating a medical condition that is treatable by the active agent.

In some embodiments, the composition may further comprise a targeting moiety attached to the self-assembled core-corona structure, which enhances the affinity of the self-assembled structure to the desired bodily site where the therapeutic activity should be exerted (e.g., a specific organ, tissue or cells).

In some embodiments, the composition may further comprise a shuttling moiety (e.g., cell penetrating peptide) attached to the self-assembled core-corona structure, which may enhance the permeability of the self-assembled structure in a specific body barrier or cell membrane.

The term "subject" (alternatively referred to herein as "patient") as used herein refers to an animal, e.g., a mammal, e.g., a human, who has been the object of treatment, observation or experiment.

The phrase "anticancer agent" or "anticancer drug", as used herein, describes a therapeutically active agent that directly or indirectly kills cancer cells or directly or indirectly inhibits, stops or reduces the proliferation of cancer cells. Anti-cancer agents include those that result in cell death and those that inhibit cell growth, proliferation and/or differentiation. In some embodiments, the anti-cancer agent is selectively toxic against certain types of cancer cells but does not affect or is less effective against normal cells. In some embodiments, the anti-cancer agent is a cytotoxic agent.

The terms "cancer" and "tumor" are used interchangeably herein to describe a class of diseases in which a group of cells display uncontrolled growth (division beyond the normal limits). The term "cancer" encompasses malignant and benign tumors as well as disease conditions evolving from primary or secondary tumors. The term "malignant tumor" describes a tumor which is not self-limited in its growth, is capable of invading into adjacent tissues, and may be capable of spreading to distant tissues (metastasizing). The term "benign tumor" describes a tumor which is non-malignant (i.e. does not grow in an unlimited, aggressive manner, does not invade surrounding tissues, and does not metastasize). The term "primary tumor" describes a tumor that is at the original site where it first arose. The term "secondary tumor" describes a tumor that has spread from its original (primary) site of growth to another site, close to or distant from the primary site.

Non-limiting examples of therapeutically active agents that may be beneficially used in embodiments of the present invention include, without limitation, one or more of an agonist agent, an amino acid agent, an analgesic agent, an antagonist agent, an antibiotic agent, an antibody agent, an antidepressant agent, an antigen agent, an antihistamine agent, an antihypertensive agent, an antiinflammatory drug, an antimetabolic agent, an antimicrobial agent, an antioxidant agent, an anti-proliferative drug, an antisense agent, a chemotherapeutic drug, an antiviral, an antiretroviral, a co-factor, a cytokine, a drug, an enzyme, a growth factor, a heparin, a hormone, an immunoglobulin, an inhibitor, a ligand, a nucleic acid, an oligonucleotide, a peptide, a phospholipid, a prostaglandin, a protein, a toxin, a vitamin and any combination thereof.

As used herein, the phrase "labeling agent" refers to a detectable moiety or a probe and includes, for example, chromophores, fluorescent compounds, phosphorescent compounds, heavy metal clusters, and radioactive labeling compounds, as well as any other known detectable moieties.

In any of the methods, uses, compositions, or products, described herein, the products described herein may be utilized in combination with additional therapeutically active agents. Such additional agents include, as non-limiting examples, chemotherapeutic agents, anti-angiogenesis agents, hormones, growth factors, antibiotics, anti-microbial agents, anti-depressants, immunostimulants, and any other agent that may enhance the therapeutic effect of the composition and/or the well-being of the treated subject.

Additional non-limiting examples of active agents include: acetylcholinesterase inhibitors, analgesics and nonsteroidal antiinflammatory agents, anthelminthics, antiacne agents, antianginal agents, antiarrhythmic agents, anti-asthma agents, antibacterial agents, anti-benign prostate hypertrophy agents, immunosuppressants, anticoagulants, antidepressants, antidiabetics, antiemetics, antiepileptics, antifungal agents, antigout agents, antihypertensive agents, antiinflammatory agents, antimalarials, antimigraine agents, antimuscarinic agents, antineoplastic agents, antiobesity agents, antiostcoporosis agents, antiparkinsonian agents, antiproliferative agents, antiprotozoal agents, antithyroid agents, antitussive agent, anti-urinary incontinence agents, antiviral agents, antiretroviral agents, anxiolytic agents, appetite suppressants, beta-blockers, cardiac inotropic agents, chemotherapeutic drugs, cognition enhancers, contraceptives, corticosteroids, Cox-2 inhibitors, diuretics, erectile dysfunction improvement agents, expectorants, gastrointestinal agents, histamine receptor antagonists, hypnotics, immunosuppressants, keratolytics, lipid regulating agents, leukotriene inhibitors, macrolides, muscle relaxants, neuroleptics, nutritional agents, opiod analgesics, protease inhibitors, sedatives, sex hormones, stimulants, vasodilators, essential fatty acids, non-essential fatty acids, proteins, peptides, sugars, vitamins, nutraceuticals, natural agents, or mixtures thereof.

In some embodiments, the active agent is hydrophobic. Hydrophobic active agents may include agents having many different types of activities.

Non-limiting examples of hydrophobic active agents include: antiproliferatives such as paclitaxel, sirolimus (rapamycin), everolimus, biolimus A9, zotarolimus, tacrolimus, and pimecrolimus and mixtures thereof; analgesics and anti-inflammatory agents such as aloxiprin, auranofin, azapropazone, benorylate, diflunisal, etodolac, fenbufen, fenoprofen calcium, flurbiprofen, ibuprofen, indomethacin, ketoprofen, meclofenamic acid, mefenamic acid, nabumetone, naproxen, oxyphenbutazone, phenylbutazone, piroxicam, sulindac; anti-arrhythmic agents such as amiodarone HCl, disopyramide, flecainide acetate, quinidine sulphate; antibacterial agents such as benethamine penicillin, cinoxacin, ciprofloxacin HCl, clarithromycin, clofazimine, cloxacillin, demeclocycline, doxycycline, erythromycin, ethionamide, imipenem, nalidixic acid, nitrofurantoin, rifampicin, spiramycin, sulphabenzamide, sulphadoxine, sulphamerazine, sulphacetamide, sulphadiazine, sulphafurazole, sulphamethoxazole, sulphapyridine, tetracycline, trimethoprim, rifampicin, bedaquiline; anti-coagulants such as dicoumarol, dipyridamole, nicoumalone, phenindione; antihypertensive agents such as amlodipine, guanethidine, benidipine, darodipine, dilitazem HCl, diazoxide, felodipine, guanabenz acetate, isradipine, minoxidil, nicardipine HCl, nifedipine, nimodipine, phenoxybenzamine HCl, prazosin HCl, reserpine, terazosin HCl; antimuscarinic agents: atropine, benzhexol HCl, biperiden, ethopropazine HCl, hyoscyamine, mepenzolate bromide, oxyphencylcimine HCl, tropicamide; antineoplastic agents and immunosuppressants such as aminoglutethimide, amsacrine, azathioprine, busulphan, chlorambucil, cyclosporin, dacarbazine, estramustine, etoposide, lomustine, melphalan, mercaptopurine, methotrexate, mitomycin, mitotane, mitozantrone, paclitaxel, procarbazine HCl, tamoxifen citrate, testolactone, topotecan, SN38, topotecan, irinotecan, exatecan, lurtotecan, imatinib, nilotinib, dasatinib, bosutinib, ponatinib; beta-blockers such as acebutolol, alprenolol, atenolol, labetalol, metoprolol, nadolol, oxprenolol, pindolol, propranolol; cardiac inotropic agents such as amrinone, digitoxin, digoxin, enoximone, lanatoside C, medigoxin; corticosteroids such as beclomethasone, betamethasone, budesonide, cortisone acetate, desoxymethasone, dexamethasone, fludrocortisone acetate, flunisolide, flucortolone, fluticasone propionate, hydrocortisone, methylprednisolone, prednisolone, prednisone, triamcinolone; lipid regulating agents such as bezafibrate, clofibrate, fenofibrate, gemfibrozil, probucol; nitrates and other anti-anginal agents such as amyl nitrate, glyceryl trinitrate, isosorbide dinitrate, isosorbide mononitrate, pentaerythritol tetranitrate; antiretrovirals such as nevirapine, efavirenz, etravirine, saquinavir, ritonavir, indinavir, lopinavir, darunavir, atazanavir, fosamprenavir, tipranavir, maraviroc, vicriviroc, and combinations thereof; antiparasitic drugs such as benznidazole, nifurtimox, nitozoxanide, miltefosine and combination thereof. Other hydrophobic active agents include, but are not limited to, active agents for treatment of hypertension (HTN), such as guanethidine.

In some embodiments, the hydrophobic active agents are new chemical entities under *in vitro* or *in vivo* preclinical and clinical evaluation.

In some embodiments, the pharmaceutical product described hereinabove are suitable to be used in the prophylaxis, diagnosis, or therapy in human or veterinary medicine for the release of biologically active cargos such as drugs, enzymes, proteins, genes, or any other agent with prophylactic, diagnostic, or therapeutic properties, or combinations of these properties.

In some embodiments of the invention, the pharmaceutical product is a topical composition formulated for administration onto the skin (including eyes, scalp, hair and nails) of a subject.

In some embodiments, the micelles are characterized by mucoadhesiveness e.g., to prolong the residence time of the PMs in the gastrointestinal track and enhance the bioavailability of the encapsulated drug.

In some embodiments, the micelles are characterized by high physical stability under unfavored conditions (e.g., extreme dilution) and upon interaction with mucosa thereby preserving the drug in the core.

In some embodiments, the micelles are characterized by a rate-controlling capacity of the drug.

In some embodiments, the micelles are in the form of micro- or nano-gel.

In some embodiments, the composition is formulated for mucosal application. As used herein, the term "mucosal application" may refer to absorption of an active agent and the like and is meant to encompass absorption across or through a mucous membrane.

The term "mucoadhesive", or any grammatical derivative thereof, as used herein refers to the phenomenon where a natural or synthetic substance applied to a mucosal epithelium, adheres, usually creating a new interface, to the mucus layer.

In the Examples section below, a test has been devised and employed which measures mucoadhesion index. Accordingly, in some embodiments, the mucoadhesion index is e.g., 0.5, 1, 2, 3, 4, 5, 6, 7, 8, 9, or 10, including any value and range therebetween.

In some embodiments, the pharmaceutical product is formulated for oral administration. In some embodiments, the pharmaceutical product is formulated for ophthalmic administration e.g., as eye drops, cream, etc. In some embodiments, the pharmaceutical composition is formulated for intranasal administration. In some embodiments, the pharmaceutical composition is formulated for inhalation. In some embodiments, the pharmaceutical composition is formulated as a pharmaceutically acceptable injectable matrix.

According to another aspect of embodiments of the present invention, there is provided the composition described herein, or pharmaceutical product comprising the composition described herein, for use in the treatment of a medical condition treatable by the therapeutically active agent. In some embodiments the medicament is formulated for oral administration.

As discussed hereinabove, a large group of drugs, e.g., chemotherapeutic agents are hydrophobic and exhibit poor solubility in aqueous solution, thus rendering their oral administration problematic. For example, many chemotherapeutic agents are typically administered intravenously. This route of administration is a major source of cost, discomfort and stress to patients, and multiple hospitalizations are required in order to complete the relatively long chemotherapeutic regimen. Thus, the enhancement of chemotherapeutic agent's solubility, by encapsulation in the herein described core-corona structure is especially beneficial and may be utilized for treating e.g., cancer and cancer metastases.

In some embodiments, the composition described hereinabove provides desirable solubility factor, as exemplified in the Examples section below.

In some embodiments, the solubility factor is solubility of active agent in the polymeric micelles divided by the intrinsic solubility of the active agent in a polymer-free medium (at 37 °C). A solubility factor of above 1 indicates that more than the amount of active agent soluble in the solvent present.

In some embodiments, the solubility factor is at least 100, least 200, least 300 least 400, least 500, least 600, least 700, least 800, least 900, least 1000, least 1200, least 1300, least 1400, or least 1500.

In some embodiments, the pharmaceutical composition described hereinabove are suitable to be used as sealants, as coatings, as lubricants and as transient barriers in the body aiming at reducing or preventing of adhesions subsequent to surgical procedures. The area of tissue engineering represents an additional important field of application of the disclosed compositions, where they can perform as the matrix for cell growth and tissue scaffolding.

The combined friction reduction and therapeutic effect is particularly advantageous when the disclosed composition is used in an application that also requires a localized enhanced effect of a therapeutically active agent, such as antimicrobial control, cell/tissue growth, regeneration and necrosis, vasodilatation/vasoconstriction, immunosuppression/immune-enhancement and the likes.

In some of any of the embodiments of the present invention, the therapeutic agent may also comprise a vasodilator to counteract vasospasm, for example an antispasmodic agent such as papaverine. The therapeutic agent may be a vasoactive agent, generally such as calcium antagonists, or alpha and beta adrenergic agonists or antagonists. In some of any of the embodiments of the present invention, the therapeutic agent may include a biological adhesive such as medical grade cyanoacrylate adhesive or fibrin glue, the latter being used to , for example, adhere an occluding flap of tissue in a coronary artery to the wall, or for a similar purpose.

In some of any of the embodiments of the present invention, the therapeutic agent may be an antibiotic agent that may be released from the core, optionally in conjunction with a controlled release carrier for persistence, to an infected organ or tissue or any other source of localized infection within the body. Similarly, the therapeutic agent may comprise steroids for the purpose of suppressing inflammation or for other reasons in a bodily site. Exemplary anti-infective agents include, for example, chlorhexidine which is added for improved biocompatibility of articles-of-manufacturing comprising the composition according to some of any of the embodiments of the present invention.

In some embodiments, compositions wherein the associating groups of the domains or monomeric units which form the self-assembled hollow structure are selected are beneficial for use in drug delivery. One exemplary use of such a composition wherein specific drug delivery is crucial is a composition for gene therapy. Such a composition can include a self-assembled hollow structure as presented herein and a combination of active agents attached to and/or encapsulated in a self-assembled hollow structure. In order to design an effective tool for local gene therapy, a chemical self-assembled core-corona structure may have the following components: a nucleic acid construct, an antisense or any other agent useful in gene therapy, for effecting the desired therapeutic effect within a cell, being attached to the self-assembled core-corona structure or being encapsulated in a self-assembled core-corona structure, that can be disassembled under physiological conditions (e.g., being biocleavable by cellular components); and a targeting agent attached to the self-assembled core-corona structure, selected to have an affinity to the desired location and optionally having a capacity for internalization into the cells at the desired location. Once reaching its designed target, the self-assembled core-corona structure is internalized into the cells and once inside the cell, the therapeutic agent is released upon interaction with cellular components that e.g., cleave either the interactions within the disclosed polymeric structure. Thus, the therapeutic agent is delivered to its final target and can exert its therapeutic activity. In some embodiments, the composition comprises a self-assembled core-corona structure as described herein and an anti-cancer drug attached thereto or encapsulated therein.

Apart from having unique, drug delivery attributes, as detailed herein, the self-assembled core-corona structures may include an active agent such as a labeling agent attached to or encapsulated therein. The encapsulation of an additional active agent to a self-assembled core-corona structure having a labeling moiety attached thereto or encapsulated therein may afford an efficient imaging probe. When the additional active agent is a targeting moiety, the encapsulation thereof to self-assembled core-corona structures having a labeling agent attached thereto or encapsulated therein, can assist in the location, diagnosis and targeting of specific loci in a host. When the additional active agent is a therapeutic agent, the encapsulation thereof to self-assembled core-corona structures having a labeling agent (e.g., indocyanine green) attached thereto or encapsulated therein, can assist in monitoring the distribution thereof in the host, thereby monitoring medical conditions as described hereinbelow. The use of the self-assembled core-corona structure as a delivery vehicle depends largely on its capacity to penetrate at least some physiological barriers and biologic degradation. To this effect, the chemical self-assembled core-corona structure can be functionalized so as to alter its surface for higher biocompatibility. Such alteration can improve the bioavailability of any other active agents attached to and/or encapsulated in the self-assembled hollow structure. Exemplary surface-active agents that can provide such bioavailability include certain polymers, which are known to exert the desired surface alteration to organic and non-organic entities.

In some embodiments, the self-assembled structure disclosed herein or at least a part thereof has mucus adhesive properties.

In some embodiments, there is provided herein a method for extending the release period in a physiological environment (e.g., blood) of at least one active agent, the method comprising encapsulating at least one active agent in the core-corona structure disclosed herein. In some embodiments, active agent is slowly released. In some embodiments, active agent is released in a controlled manner. In some embodiments, active agent is identified as being unstable in the physiological environment. In this context, the term "controlled manner" indicates that the drug is released substantially constantly, or in accordance with a pre-defined rate to e.g., a target cell. Herein, the term "constantly" may refer to a time duration of about e.g., 30 min, 1 h, 2 h, 3 h, 4 h, 5 h, 6 h, 7 h, 8 h, 9 h, 10 h, 11 h, 12 h, 13 h, 14 h, 15 h, 16 h, 17 h, 18 h, 19 h, 20 h, 21 h, 22 h, 23 h, 24 h, 2 days, 3 days, 4 days, 5 days, 6 days, 7 days, 8 days, 9 days, 10 days, 11 days, 12 days, 13 days, 14 days, 15 days, 16 days, 17 days, 18days, 19 days, 20 days, 21 days, 22 days, 23 days, 24 days, 25 days, 26 days, 27 days, 28 days, 29 days, 30 days, including any value therebetween.

In some embodiments, the pre-defined rate may be affected by the non-covalent crosslinking density (i.e., the number of non-covalent cross-links).

In some embodiments, the disclosed composition is enabled to encapsulate therein active substance *via* a variety of interactions depending on the intended use of the desired release characteristics of the active substance, the desired surface properties of the object and many more.

In some embodiments, the composition disclosed herein is designed as uniform and adherent coating and may further be designed to controllably release active substances that are encapsulated therein.

### *Method of Treatments*

According to some embodiments, there is provided a method of monitoring medical conditions such as, but are not limited to, cancer and infections (e.g., viral infection, or bacterial infection). Any references to methods of treatment by therapy or surgery or in vivo diagnosis methods of this description is to be interpreted as a reference to compounds, pharmaceutical compositions and medicaments of the present invention for use in those methods.

It is to note that herein, by targeting a therapeutically active agent *via* the methodologies described herein, the toxicity of the therapeutically active agent is substantially reduced both systemically and locally in body sites where the active agent is not expected to exert its activity. Consequently, besides the use of the amphiphilic polymers described herein in a clinically evident disease, optionally in combination with other drugs, these amphiphilic polymers may potentially be used as a long term-prophylactic for individuals who are at risk for relapse due to residual dormant cancers.

As further detailed hereinabove, the term "cancer" or "cancer cells" describes a group of cells which display uncontrolled growth (division beyond the normal limits).

As described hereinabove, cancers treatable by the compositions described herein include, but are not limited to, solid, including carcinomas, and non-solid, including hematologic malignancies.

As used herein, the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating", or any grammatical derivative thereof, is meant to refer to abrogating, substantially inhibiting, slowing or reversing the progression of a condition, substantially ameliorating clinical or aesthetical symptoms of a condition or substantially preventing the appearance of clinical or aesthetical symptoms of a condition.

It is understood that the composition of the present invention may be administered in conjunction with other drugs, including other antiviral and/or anti-cancer drugs.

As further described hereinabove, the composition of the invention may comprise a labeling agent. Composition comprising a labeling agent may be used in suitable imaging techniques.

Suitable imaging techniques include but are not limited to positron emission tomography (PET), gamma-scintigraphy, magnetic resonance imaging (MRI), functional magnetic resonance imaging (fMRI), magnetoencephalography (MEG), single photon emission computerized tomography (SPECT) computed axial tomography (CAT) scans, ultrasound, fluoroscopy and conventional X-ray imaging.

The choice of an appropriate imaging technique depends on the nature of the labeling agent, and is within the skill in the art. For example, if the labeling agent comprises Gd ions, then the appropriate imaging technique is MRI; if the labeling agent comprises radionuclides, an appropriate imaging technique is gamma-scintigraphy; if the labeling agent comprises an ultrasound agent, ultrasound is the appropriate imaging technique; if the labeling agent comprise a near infrared (NIR) dye, NIR is the appropriate technique; etc.

*The Process*

According to another aspect of embodiments of the present invention, there is provided a process of preparing the amphiphilic graft polymer being in form of closed core-corona and self-assembled structure described herein, the closed hollow and self-assembled structure further comprising one or more active agents, the process comprising the steps of:

grafting a hydrophobic polymeric backbone to a hydrophilic polymeric backbone in a solution, thereby forming an amphiphilic graft polymer configured to form a hydrophobic core and a hydrophilic corona;

dispersing the amphiphilic graft copolymer in aqueous medium at a concentration above a predefined minimal concentration;

adding an active agent to the solution e.g., as solid or in a organic solvent that is water-miscible, thereby encapsulating the active agent within the hydrophobic core;

optionally, eliminating the organic solvent by evaporation or dialysis;

adding a non-covalent cross-linker to the solution, so as to form a non-covalent layer deposited on at least portion of the hydrophilic corona, thereby forming an amphiphilic graft polymer being in form of closed core-corona and self-assembled structure further encapsulating one or more active agents; and

optionally drying the suspension to produce a dry powder.

In some embodiments, the predefined concentration is critical micelle concentration (CMC).

In some embodiments, the process further comprises a step of heating an aqueous solution containing said amphiphilic graft polymer to a temperature that ranges from about 30°C to about 50°C (e.g., 37 °C) prior to the step of adding the active agent to the solution.

In some embodiments, the process further comprises a step of cooling the solution to a temperature lower than 30°C, thereby stabilizing the amphiphilic graft polymer.

In some embodiments, the process further comprises a step of diluting the dispersion to a final concentration below the predefined concentration, following the step of adding an active agent to the dispersion thereby stabilizing the amphiphilic graft polymer.

Herein, the step of polymerizing the hydrophobic component may be performed by any polymerization method described hereinabove, for example by subjecting the corresponding monomers to conditions that allow them to associate therebetween *via* their associating groups.

Herein, the active agent may be any active agent described hereinabove.

In some embodiments, the step of adding a non-covalent crosslinker to the solution is performed prior to the step of adding an active agent to the solution.

In some embodiments, the step of adding a non-covalent crosslinker to the solution is performed after the step of adding an active agent to the solution.

In some embodiments the process described herein, is such wherein the concentration of the active agent in the solution and the concentration of the amphiphilic graft polymer in the aqueous solution are selected so as to obtain a pre-determined molar ratio of the active agent to the amphiphilic graft polymer.

The construction of the desired amphiphilic graft polymer, comprising the encapsulated active agent may be verified by techniques known in the art.

Examples of such techniques are detailed in the Examples section and include, zeta-potential (Z-potential) measurements, DLS, NTA, solubility factors of the active agents, visual inspection, etc.

*General*

As used herein the term "about" refers to $\pm$ 10 %.

The terms "comprises", "comprising", "includes", "including", "having" and their conjugates mean "including but not limited to". The term "consisting of" means "including and limited to". The term "consisting essentially of" means that the composition, method or structure may include additional ingredients, steps and/or parts, but only if the additional ingredients, steps and/or parts do not materially alter the basic and novel characteristics of the claimed composition, method or structure.

The word "exemplary" is used herein to mean "serving as an example, instance or illustration". Any embodiment described as "exemplary" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment of the invention may include a plurality of "optional" features unless such features conflict.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

In those instances where a convention analogous to "at least one of A, B, and C, etc." is used, in general such a construction is intended in the sense one having skill in the art would understand the convention (*e.g.*, "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense that one having skill in the art would understand the convention (*e.g.*, "a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

As used herein, the term "alkyl" describes an aliphatic hydrocarbon including straight chain and branched chain groups. The alkyl group has 1 to 100 carbon atoms, and more preferably 1-50 carbon atoms. Whenever a numerical range; e.g., "1-100", is stated herein, it implies that the group, in this case the alkyl group, may contain 1 carbon atom, 2 carbon atoms, 23 carbon atoms, etc., up to and including 100 carbon atoms. In the context of the present invention, a "long alkyl" or "high alkyl" is an alkyl having at least 10, or at least 15 or at least 20 carbon atoms in its main chain (the longest path of continuous covalently attached atoms), and may include, for example, 10-100, or 15-100 or 20-100 or 21-100, or 21-50 carbon atoms. A "short alkyl" or "low alkyl" has 10 or less main-chain carbons. The alkyl can be substituted or unsubstituted, as defined herein.

The term "alkyl", as used herein, also encompasses saturated or unsaturated hydrocarbon, hence this term further encompasses alkenyl and alkynyl.

The term "alkenyl" describes an unsaturated alkyl, as defined herein, having at least two carbon atoms and at least one carbon-carbon double bond. The alkenyl may be substituted or unsubstituted by one or more substituents, as described hereinabove.

The term "alkynyl", as defined herein, is an unsaturated alkyl having at least two carbon atoms and at least one carbon-carbon triple bond. The alkynyl may be substituted or unsubstituted by one or more substituents, as described hereinabove.

The term "cycloalkyl" or "cycloalkane" describes an all-carbon monocyclic or fused ring (*i.e.,* rings which share an adjacent pair of carbon atoms) group where one or more of the rings does not have a completely conjugated pi-electron system. The cycloalkyl group may be substituted or unsubstituted, as indicated herein.

The term "aryl" or "aromatic" describes an all-carbon monocyclic or fused-ring polycyclic (*i.e.*, rings which share adjacent pairs of carbon atoms) groups having a completely conjugated pi-electron system. The aryl group may be substituted or unsubstituted, as indicated herein.

The term "alkoxy" describes both an -O-alkyl and an -O-cycloalkyl group, as defined herein.

The term "aryloxy" describes an -O-aryl, as defined herein.

Each of the alkyl, cycloalkyl and aryl groups in the general formulas herein may be substituted by one or more substituents, whereby each substituent group can independently be, for example, halide, alkyl, alkoxy, cycloalkyl, alkoxy, nitro, amine, hydroxyl, thiol, thioalkoxy, thiohydroxy, carboxy, amide, aryl and aryloxy, depending on the substituted group and its position in the molecule. Additional substituents are also contemplated.

The term "halide", "halogen" or "halo" describes fluorine, chlorine, bromine or iodine.

The term "haloalkyl" describes an alkyl group as defined herein, further substituted by one or more halide(s).

The term "hydroxyl" or "hydroxy" describes a -OH group.

The term "thiohydroxy" or "thiol" describes a -SH group.

The term "thioalkoxy" describes both an -S-alkyl group, and a -S-cycloalkyl group, as defined herein.

The term "thioaryloxy" describes both an -S-aryl and a -S-heteroaryl group, as defined herein.

The term "amine" describes a -NR'R" group, with R' and R" as described herein.

The term "heteroaryl" describes a monocyclic or fused ring (*i.e.,* rings which share an adjacent pair of atoms) group having in the ring(s) one or more atoms, such as, for example, nitrogen, oxygen and sulfur and, in addition, having a completely conjugated pi-electron system. Examples, without limitation, of heteroaryl groups include pyrrole, furane, thiophene, imidazole, oxazole, thiazole, pyrazole, pyridine, pyrimidine, quinoline, isoquinoline and purine.

The term "heteroalicyclic" or "heterocyclyl" describes a monocyclic or fused ring group having in the ring(s) one or more atoms such as nitrogen, oxygen and sulfur. The rings may also have one or more double bonds. However, the rings do not have a completely conjugated pi-electron system. Representative examples are piperidine, piperazine, tetrahydrofuran, tetrahydropyrane, morpholino and the like.

The term "carboxy" or "carboxylate" describes a -C(=O)-OR' group, where R' is hydrogen, alkyl, cycloalkyl, alkenyl, aryl, heteroaryl (bonded through a ring carbon) or heteroalicyclic (bonded through a ring carbon) as defined herein.

The term "carbonyl" describes a -C(=O)-R' group, where R' is as defined hereinabove.

The above-terms also encompass thio-derivatives thereof (thiocarboxy and thiocarbonyl).

The term "thiocarbonyl" describes a -C(=S)-R' group, where R' is as defined hereinabove.

A "thiocarboxy" group describes a -C(=S)-OR' group, where R' is as defined herein.

A "sulfinyl" group describes an -S(=O)-R' group, where R' is as defined herein.

A "sulfonyl" or "sulfonate" group describes an -S(=O)$_2$-R' group, where Rx is as defined herein.

A "carbamyl" or "carbamate" group describes an -OC(=O)-NR'R" group, where R' is as defined herein and R" is as defined for R'.

A "nitro" group refers to a -NO$_2$ group.

A "cyano" or "nitrile" group refers to a -C=N group.

As used herein, the term "azide" refers to a -N$_3$ group.

The term "sulfonamide" refers to a -S(=O)$_2$-NR'R" group, with R' and R" as defined herein.

The term "phosphonyl" or "phosphonate" describes an -O-P(=O)(OR')$_2$ group, with R' as defined hereinabove.

The term "phosphinyl" describes a -PR'R" group, with R' and R" as defined hereinabove.

The term "alkaryl" describes an alkyl, as defined herein, which substituted by an aryl, as described herein. An exemplary alkaryl is benzyl.

The term "heteroaryl" describes a monocyclic or fused ring (*i.e.,* rings which share an adjacent pair of atoms) group having in the ring(s) one or more atoms, such as, for example, nitrogen, oxygen and sulfur and, in addition, having a completely conjugated pi-electron system. Examples, without limitation, of heteroaryl groups include pyrrole, furane, thiophene, imidazole, oxazole, thiazole, pyrazole, pyridine, pyrimidine, quinoline, isoquinoline and purine. The heteroaryl group may be substituted or unsubstituted by one or more substituents, as described hereinabove. Representative examples are thiadiazole, pyridine, pyrrole, oxazole, indole, purine and the like.

As used herein, the terms "halo" and "halide", which are referred to herein interchangeably, describe an atom of a halogen, that is fluorine, chlorine, bromine or iodine, also referred to herein as fluoride, chloride, bromide and iodide.

The term "haloalkyl" describes an alkyl group as defined above, further substituted by one or more halide(s).

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

## EXAMPLES

Reference is now made to the following examples which, together with the above descriptions, illustrate the invention in a non-limiting fashion.

*Materials*

The synthesis of the different copolymers is presented in the following examples.

In general, low molecular weight ($M_w$) chitosan (CS, $M_w$ of 50 kg/mole, degree of deacetylation of 94%, Glentham Life Science, Corsham, UK), poly(vinyl alcohol) (PVA, $M_w$, of 31 kg/mole), N-isopropylacrylamide (NiPAam, 99% stabilized with 500 ppm MEHQ, J&K, Beijing, China; recrystallized from n-hexane before use), cerium ammonium nitrate (CAN, STREM Chemicals, Newburyport, MA, USA), tetramethylethylenediamine (TEMED, Alfa Aesar, Heysham, UK), hydroxyquinone (HQ, Merck, Hohenbrunn, Germany), nitric acid 70% (Bio-lab, Jerusalem, Israel) were used as received. Methyl methacrylate (MMA, Alfa Aesar) was distilled under vacuum before use to remove inhibitor. All the other solvents were of analytical or spectroscopic grade and purchased from Bio-lab or Gadot (Netanya, Israel) and used without further purification.

**EXAMPLE 1**

**Synthesis and characterization of CS-g-oligo(NiPAam) copolymers**

In exemplary procedures, CS (0.4 g) was dissolved in degassed nitric acid aqueous solution (100 mL, 0.05 M) overnight. Then, an aqueous solution of TEMED (0.18 mL) in water (50 mL) was degassed, poured into the CS solution and purged with nitrogen (30 min). The solution was heated to 35°C and a NiPAam aqueous solution (48 mL, containing 0.4-1.6 g NiPAam) added. Finally, an aqueous solution of CAN (2 mL, 0.66 g) was added. After 6 h of reaction under magnetic stirring, HQ was added to quench the reaction. Reaction crudes were thoroughly dialyzed against distilled water (regenerated cellulose dialysis membranes; MWCO of 12,000-14,000 g/mol, Spectra/Por® 4 nominal flat width of 75 mm, diameter of 48 mm, and volume/length ratio of 18 mL/cm; Spectrum Laboratories, Inc., Rancho Dominguez, CA, USA) for 48 h, frozen (liquid $N_2$, 5 min) and freeze-dried (72-96 h, Labconco Free Zone 4.5 plus L Benchtop Freeze Dry System with collector temperature of -85°C, Labconco, Kansas City, MO, USA). All the copolymers were characterized by proton nuclear magnetic resonance ($^1$H-NMR, 400-MHz Bruker® Avance III High Resolution spectrometer, Bruker BioSpin GmbH, Rheinstetten, Germany), Fourier-Transform infrared spectroscopy (FTIR, Equinox 55 spectrometer, Bruker optics Inc., Ettlingen, Germany; using KBr windows) and differential scanning calorimetry (DSC, DSC 2 STAR[e] system simultaneous thermal analyzer using the STAR[e] Software V13 (Metter-Toledo; Schwerzenbach, Switzerland, with intracooler Huber TC100 under dry $N_2$ atmosphere) and gel permeation chromatography (GPC, Agilent 1100 Series HPLC System, Agilent Technologies, Santa Clara, CA, USA with water as eluent and refractive index detector). The weight percent of NiPAam (%NiPAam) in the different CS-g-oligo(NiPAam) copolymers was determined by $^1$H-NMR using a calibration curve built with CS:NiPAam physical mixtures and summarized in Table 1.

The percent of grafting (% Grafting) was calculated according to the following Equation:

$$\% \text{ Grafting} = \%\text{NiPAam}/(100 - \%\text{NiPAam}) \text{ x } 100$$

The hydrophilic-lipophilic balance (HLB) was estimated by a modification the Griffin's method according to the following Equation:

$$\text{HLB} = 20 \text{ x } (M_h/M)$$

where $M_h$ is the weight content of the hydrophilic component and M is the total weight of copolymer. For this purpose, CS was considered a non-ionic polymer.

In general, the copolymers are named as CS-NX, where X is the weight percent (% w/w) of NiPAam in the molecule, as determined by $^1$H-NMR.

Table 1 below summarizes the weight percent of NiPAam (% w/w) (referred to as: "NX" where X represents the corresponding percent of the NiPAam in the composition, for example, N36 denotes 36% NiPAam) in the CS-graft copolymers and the grafting efficiency as determined by $^1$H-NMR (Figure 1A). "g" denotes grafted

**Table 1**

| CS-*g*-oligo(NiPAam) copolymer | %NiPAam | % Grafting | HLB |
|---|---|---|---|
| CS-N36 | 36 | 56 | 12.8 |
| CS-N52 | 52 | 108 | 9.6 |
| CS-N62 | 62 | 163 | 7.6 |

(continued)

| CS-*g*-oligo(NiPAam) copolymer | %NiPAam | % Grafting | HLB |
|---|---|---|---|
| CS-N74 | 74 | 285 | 5.2 |

**Elemental analysis:** The NiPAam content was confirmed by elemental analysis of pristine CS and the corresponding copolymers. Samples (2-3 mg) were mixed with 8-10 mg of V in Sn. The combustion temperature was 950°C, the carrier gas (He, 99.999% purity) was injected at a flow rate of 140 mL/min, and $O_2$ was added for 5 s in a flow rate of 250 mL/min. The standards used for calibration were cystine, 2,5-(bis(5-tert-butyl-2-benzo-oxazol-2-yl) thiophene, sulfanilamide and methionine. The %NiPAam in the copolymers was calculated according to the following Equation.

$$\%NiPAam = \frac{\%N_{Total} - F_{CS} \times \%N_{CS}}{\%N_{NiPAam}} \times 100$$

where $\%N_{Total}$ and $\%N_{CS}$ are average N contents (%) in the copolymer and pristine CS, respectively, as determined by elemental analysis, $\%N_{NiPAam}$ is the theoretical N content (%) in pure NiPAam (12.2%) and $F_{CS}$ is the weight fraction of CS in the copolymer as calculated by $^1$H-NMR

The analysis confirmed the gradual increase of the %NiPAam content, values being between 39% (64% grafting) and 69% (223% grafting). For a NiPAam:CS feeding ratio of 1, the reaction yield was 36%, probably due to the presence of substantial amounts of very low molecular weight residues in pristine CS, as confirmed by dialysis, where only approximately 50-75% of the original CS sample was recovered. The yield for a feeding ratio of 2 increased to 65% due to the increase of the molecular weight in the side-chain that prevented the loss of the molecule out of the dialysis membrane. Higher feeding ratio of 3 and 4 has led to a decrease of the yield to 54% and 37%, respectively. These results strongly suggested that for greater NiPAam:CS feeding ratios, NiPAam underwent a more significant homopolymerization; low molecular weight homopolymer was eliminated by dialysis as explained in the previous section. The synthesis of CS-g-oligo(NIPAam) block copolymers was also characterized by FTIR (**Figure 1B**). Pure CS displayed typical absorption bands at 3456 cm[-1] (O-H and N-H stretching), 2919 cm[-1] (C-H stretching), 1535 cm[-1] (N-H bending), 1383 cm[-1] (amide III), 1157 cm[-1] (C-O-C stretching in the glycosidic bonds) and 1053 cm[-1] (skeletal vibration involving the COO stretching) (**Figure 1B**). A band at 1641 cm[-1] of N-acetyl moiety confirmed that the CS was only partly deacetylated. Graft copolymers exhibited strong characteristic bands of poly(NiPAam) at 1545 cm[-1] (bending of N-H of amide group) and several of CS detailed before, including a strong characteristic CS band at 1383 cm[-1]. As observed, a gradual increase of the %NiPAam led to an increase of the relative intensity of the band at 1545 cm-1 with respect to the band at 1383 cm[-1] assigned to CS, as exemplified for CS-N52 and CS-N62 (**Figure 1B**). The stretching of C=O of NiPAam at 1626-1640 cm[-1] partly overlapped with that of CS though it also gradually increased for higher %NiPAam as a single broader band at 1641 cm[-1].

*Molecular weight* Number- and weight-average molecular weights ($M_n$ and $M_w$, respectively) and molecular weight polydispersity index ($M_w/M_n$) were determined by gel permeation chromatography (GPC, Agilent 1100 Series HPLC System, Agilent Technologies, Santa Clara, CA, USA) with refractive index detector and a Novema 1000 column (300 $\times$ 8 mm, NH-functionalized acrylate copolymer network particle size of 10 $\mu$m, PSS Polymer Standards Service GmbH, Mainz, Germany). Sample concentrations were 0.1% in the mobile phase (water containing 0.3M formic acid). Runs were conducted with injection flow of 1 mL/min, at 20°C. Poly(vinylpyrrolidone) standards were used for $M_w$ calibration and the calculation done with Win-GPC UniChrom Software (PSS Polymer Standards Service). In addition, the viscosity average molecular weight ($M_v$) of one derivative (CS-N52) was determined by capillary viscometry. For this, the relative viscosity of copolymer solutions of concentration between 0.006-0.1% w/v in 0.3 M acetic acid/0.2 M sodium acetate aqueous buffer was measured with a Cannon Fenske Viscometer No. 25 (CANNON Instrument Co., State College, PA, USA), at 25°C. The viscosity molecular weight was then calculated using the Mark-Houwink equation ([η] = $K \times M^a$) and constants K = 7.6 $\times$ 10[-4] dL/g and $a$ = 0.76.

The obtained copolymers molecular weight was evaluated and compared to the pristine CS. The latter displayed $M_n$ and $M_w$ of 3,400 and 17,300 g/mole, respectively, and $M_w/M_n$ of 5.08. For the copolymers, similar molecular weights with $M_n$ and $M_w$ between 4,650 and 5856 g/mole and 11,300 and 21,200 g/mole, respectively, and $M_w/M_n$ in the 2.43-3.62 range were measured. The NiPAam grafting is supposed to increase the molecular weight. Moreover, increase of the feeding ratio is followed by the copolymers %NiPAam increase, thus the obtained results here could be caused by a very slight decrease of the molecular weight of the CS backbone with respect to the pristine polysaccharide, probably due to partial hydrolytic depolymerization of glycosidic bonds. At the same time, the different chemical nature and architecture of these copolymers with respect to the GPC standard might also affect the results to some extent. The GPC is known as a reliable method for determination of linear polymer molecular weight whereas for other cases a distorted pictured might be obtained. Nevertheless, this method was used for primary estimation. Then in order to support these data, the molecular weight of CS-N52 was also measured by viscosity (**Figure 1C**).

The intrinsic viscosity [η] was extracted by the limit of the relative viscosity as the polymer solute concentration approached to zero. Then, the $M_v$ was determined using the Mark-Houwink equation. The obtained Mv was 15,000 g/mole and it was in very good agreement with GPC data. This result indicated that despite some deviations, the results measured by GPC were reliable.

**Thermal analysis**: The thermal behavior of the different copolymers was analyzed employing a DSC 2 STAR[e] system simultaneous thermal analyzer with STAR[e] Software V13 (Metter-Toledo, Schwerzenbach, Switzerland) with intra-cooler (Huber TC100) under dry $N_2$ atmosphere (flow of 20 mL/min) and In as standard. Samples (2.0-2.5 mg) were sealed in 40 μL Al-crucibles pans (Metter-Toledo) and subjected to the following treatments at a heating/cooling rate of $20^0$C/min: (i) 25 to $100^0$C (first heating ramp to erase the thermal history), (ii) $100^0$C for 30 min (isotherm in order to evaporate water residues), (iii) $100^0$C to $200^0$C (continue heating ramp), (iv) 200°C to 10°C (cooling ramp) and (v) 10°C to 200°C (second heating ramp). The glass transition temperature ($T_g$) of oligo(NiPAam) blocks was determined in the second heating ramp.

Two possible thermal transitions could be expected for the copolymers, glass transition ($T_g$) of oligo(NiPAam) blocks and decomposition of CS. The latter occurs at much higher temperatures than the working range and thus, it could not be revealed. Conversely, all the copolymers showed the characteristic $T_g$ of poly(NiPAam) within in a very narrow temperature range between 139 and 142 °C, except for CS-N36 where no transition was observed probably for the low % NiPAam in the copolymer. In addition, the greater the %NiPAam, the higher the $T_g$. The thermal behavior of pristine CS, CS-N52 and CS -N62 is presented in **Figure 1D.**

## EXAMPLE 2

### Synthesis of PVA-g-oligo(NiPAam) copolymers

In exemplary procedures, PVA (0.4 g) was dissolved in degassed nitric acid aqueous solution (100 mL, 0.05 M) overnight. Then, an aqueous solution of TEMED (0.18 mL) in water (50 mL) was degassed, poured into the CS solution and purged with nitrogen (30 min). The solution was heated to 35°C and a NiPAam aqueous solution (48 mL, containing 0.4-1.6 g NiPAam) added. Finally, an aqueous solution of CAN (2 mL, 0.66 g) was added. After 6 h of reaction under magnetic stirring, HQ was added to quench the reaction. Reaction crudes were thoroughly dialyzed against distilled water (regenerated cellulose dialysis membranes; MWCO of 12,000-14,000 g/mol, Spectra/Por® 4 nominal flat width of 75 mm, diameter of 48 mm, and volume/length ratio of 18 mL/cm) for 48 h, frozen (liquid $N_2$, 5 min) and freeze-dried (72-96 h). All the copolymers were characterized by $^1$H-NMR, FTIR and DSC. The weight percent of NiPAam (%NiPAam) in the different PVA-g-oligo(NiPAam) copolymers was determined by $^1$H-NMR using a calibration curve built with PVA:NiPAam physical mixtures and summarized in Table 2. The percent of grafting and the HLB were calculated as described hereinabove.

In general, the copolymers are named as PVA-NX, where X is the weight percent (% w/w) of NiPAam in the molecule.

Table 2 below summarizes the weight percent of NiPAam (% w/w) in the PVA-graft copolymers and grafting efficiency as determined by $^1$H-NMR.

<div align="center">

**Table 2**

| PVA-*g*-oligo(NiPAam) copolymer | % NiPAam | % Grafting | HLB |
|---|---|---|---|
| PVA-N23 | 23 | 30 | 14.4 |
| PVA-N40 | 340 | 67 | 12.0 |
| PVA-N56 | 56 | 127 | 8.8 |
| PVA-N68 | 68 | 213 | 6.4 |

</div>

## EXAMPLE 3

### Synthesis of CS-g-poly(methyl methacrylate) (CS-g-PMMA) copolymers

In exemplary procedures, CS (0.4 g) was dissolved in degassed nitric acid aqueous solution (100 mL, 0.05 M) overnight. Then, an aqueous solution of TEMED (0.18 mL) in water (50 mL) was degassed, poured into the CS solution and purged with nitrogen (30 min). The solution was heated to 35 °C and a MMA aqueous solution (48 mL, containing 0.4-1.6 g MMA) was added. Finally, an aqueous solution of CAN (2 mL, 0.66 g) was added. After 3 h of reaction under magnetic stirring, HQ was added to quench the reaction. Reaction crudes were thoroughly dialyzed against distilled water (regenerated cellulose dialysis membranes; MWCO of 12,000-14,000 g/mol, Spectra/Por® 4 nominal flat width of 75 mm, diameter of 48 mm, and volume/length ratio of 18 mL/cm) for 48 h, frozen (liquid $N_2$, 5 min) and freeze-dried (72-96 h). All the copolymers were characterized by $^1$H-NMR, FTIR and DSC. The weight percent of MMA (% MMA) in the different CS-g-PMMA copolymers was determined by $^1$H-NMR using a calibration curve built with CS:MMA physical mixtures and summarized in Table 3 below. The percent of grafting (% Grafting) was calculated as described hereinabove.

In general, the copolymers are named as CS-PMMAX, where X is the weight percent (% w/w) of MMA in the molecule.

Table 3 below summarizes the weight percent of MMA (% w/w) in the CS-graft copolymers and grafting efficiency as determined by $^1$H-NMR.

**Table 3**

| CS-*g*-PMMA copolymer | %PMMA | % Grafting | HLB |
|---|---|---|---|
| CS-PMMA51 | 51 | 104 | 9.8 |
| CS-PMMA75 | 75 | 300 | 5.0 |

## EXAMPLE 4

### Micellization of CS-g-oligo(NiPAam) copolymers

In exemplary procedures, stock aqueous solutions (1% w/v in buffer acetate pH 5) of the different copolymers were diluted (0.0001-1% w/v) in the same medium and stabilized at 37°C for at least 24 h. Then, the intensity of the scattered light (DCR) expressed in kilo counts per second (kcps) was measured by Dynamic Light Scattering (DLS, Zetasizer Nano-ZS, Malvern Instruments, Malvern, UK) provided with a 4 mW He-Ne laser ($\lambda$ = 633 nm), a digital correlator ZEN3600 and a Non-Invasive Back Scatter (NIBS®) technology (37°C) and plotted as a function of the copolymer concentration (% w/v). Measurements were carried out at a scattering angle of 173° to the incident beam and data were analyzed using CONTIN algorithms (Malvern Instruments). Data for each single specimen was the result of at least six runs. The micellization was observed as a sharp increase in the scattering intensity and the intersection between the two straight lines corresponded to the critical micellar concentration (CMC).

Table 4 below presents the CMC values of the different CS-g-oligo(NiPAam) copolymers at 37°C. No CMC was detected at 25°C.

**Table 4**

| CS-*g*-oligo(NiPAam) copolymer | CMC ($10^{-3}$ % w/v) | CMC (mg/mL) |
|---|---|---|
| CS-N36 | 40.0 | 0.40 |
| CS-N52 | 6.6 | 0.07 |
| CS-N62 | 4.0 | 0.04 |
| CS-N74 | 2.0 | 0.02 |

## EXAMPLE 5

### Micellization of PVA-g-oligo(NiPAam) copolymers

The same procedure described in EXAMPLE 4 was applied.

Table 5 below presents the CMC values of the different PVA-g-oligo(NiPAam) copolymers at 37°C. No CMC was detected at 25°C.

**Table 5**

| PVA-*g*-oligo(NiPAam) copolymer | CMC ($10^{-3}$ % w/v) | CMC (mg/mL) |
|---|---|---|
| PVA-N23 | 111.0 | 1.1 |
| PVA-N40 | 35.0 | 0.35 |
| PVA-N56 | 30.0 | 0.30 |
| PVA-N68 | 5.3 | 0.053 |

## EXAMPLE 6

### *Micellization of CS-g-PMMA copolymers*

The same procedure described in EXAMPLE 4 was applied.

Table 6 below presents the CMC values of the different CS-g-PMMA copolymers at 25°C and 37°C.

**Table 6**

| CS-g-PMMA copolymer | 25°C | | 37°C | |
|---|---|---|---|---|
| | CMC ($10^{-3}$ % w/v) | CMC (mg/mL) | CMC ($10^{-3}$ % w/v) | CMC (mg/mL) |
| CS-PMMA51 | 0.70 | 0.007 | 0.4 | 0.004 |
| CS-PMMA75 | 1.0 | 0.01 | 1.0 | 0.01 |

## EXAMPLE 7

### *Preparation of non-crosslinked CS-g-oligo(NiPAam) micelles*

In exemplary procedures, aqueous solutions of the different copolymers in a range of concentrations (from CMC to 5% w/v) were prepared by simple dissolution in a diluted acetic acid solution of pH 5. To characterize the hydrodynamic diameter ($D_h$), the size distribution (polydispersity index, PDI) and the Z-potential (Z-potential), of 1% w/v polymeric micelles were analyzed using the Zetasizer Nano-ZS, at 37°C. For Z-potential, laser Doppler micro-electrophoresis in the same instrument was used.

Table 7 below presents representative sizes, $D_h$, (in nm), size distributions (PDI) and Z-potential values of 1% w/v of CS-g-oligo(NiPAam) copolymers at pH 5 and 37°C.

**Table 7**

| CS-g-oligo(NiPAam) copolymer | $D_h$ (nm) | PDI | Z-potential (mV) |
|---|---|---|---|
| CS-N36 | 436 | 0.142 | +23 |
| CS-N52 | 345 | 0.109 | +22 |
| CS-N62 | 330 | 0.100 | +16 |
| CS-N74 | 336 | 0.056 | +7 |

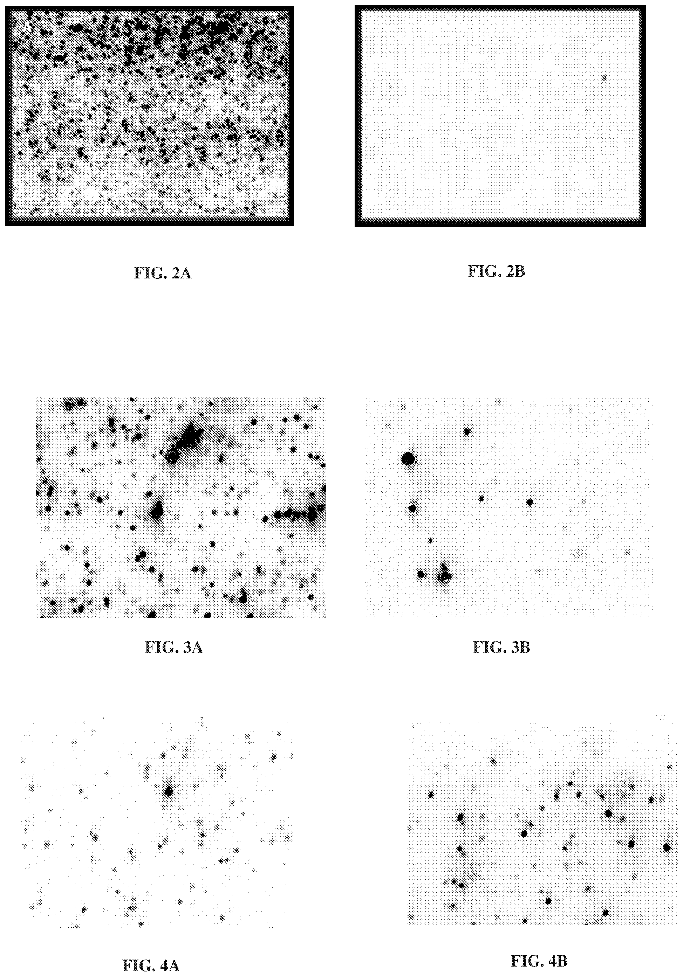

In addition, 0.08% w/v polymeric micelles were visualized by Nanoparticle Tracking Analysis (NTA, NS500-Zeta HSB system with high sensitivity camera and red laser of 638 nm for fluorescent analysis; Malvern Instruments), at 37 and 25°C. At 37°C, polymeric micelles were stable due to the hydrophobic behaviour of oligo(NiPAam) blocks **(Figure 2A)**. Conversely, at 25°C, almost no micelles could be observed by NTA, confirming the massive disassembly of the polymeric micelles upon storage at 25°C **(Figure 2B)**.

## EXAMPLE 8

### *Preparation of non-crosslinked PVA-g-oligo(NiPAam) micelles*

The same procedure of EXAMPLE 7 was applied.

Table 8 below presents representative sizes $D_h$, size distributions (PDI), and Z-potential values of 1% w/v polymeric micelles of PVA-g-oligo(NiPAam) copolymers, at 37 °C.

**Table 8**

| PVA-*g*-oligo(NiPAam) copolymer | $D_h$ (nm) | PDI | Z-potential (mV) |
|---|---|---|---|
| PVA-N23 | 253 | 0.12 | -3 |
| PVA-N40 | 315 | 0.13 | +4 |
| PVA-N56 | 244 | 0.06 | -12 |
| PVA-N68 | 273 | 0.19 | -6 |

In addition, 0.05% w/v polymeric micelles were visualized by NTA, at 37 and 25°C. At 37 °C, polymeric micelles were stable due to the hydrophobic behaviour of oligo(NiPAam) blocks **(Figure 3A).** Conversely, at 25°C, a sharp decrease in the concentration of micelles was observed by NTA, confirming the substantial disassembly of the non-crosslinked polymeric micelles upon storage at 25°C **(Figure 3B).**

### EXAMPLE 9

### Preparation of non-crosslinked PVA-g-oligo(NiPAam) micelles

The same procedure of EXAMPLE 7 was followed.

Table 9 below presents representative sizes $D_h$, size distributions (PDI), and Z-potential values of 1% w/v polymeric micelles of CS-g-PMMA micelles, at 25 and 37°C.

**Table 9**

| CS-g-PMMA copolymer | 25°C | | | 37°C | | |
|---|---|---|---|---|---|---|
| | $D_h$ (nm) | PDI | Z-potential (mV) | $D_h$ (nm) | PDI | Z-potential (mV) |
| CS-PMMA51 | 257 | 0.41 | +36 | 198 | 0.22 | +36 |
| CS-PMMA75 | 828 | 0.48 | +21 | 936 | .069 | +22 |

### EXAMPLE 10

### Non-covalent corona crosslinking of CS-N52 micelles

In exemplary procedures, CS-N52 micelles (0.008% w/v) were stabilized at 37°C for at least 24 h. Then, sodium tripolyphosphate (TPP, Sigma-Aldrich, St. Louis, MO, USA) solution (1% w/v; volumes of 1-5 μL TPP per mL of polymeric solution) was added at 37°C, the system stored for at least 6 h to allow the ionotropic crosslinking of the micellar corona and visualized by NTA **(Figure 4A).** To confirm the stability of the crosslinking, polymeric micelles were stored at 25°C for 12 h and visualized again by NTA. The concentration of the micelles remained almost unchanged **(Figure 4B).**

Table 10 below presents the particle size and concentration of non-covalently crosslinked CS-N52 polymeric micelles (0.008% w/v) as determined by DLS and NTA, at room temperature.

**Table 10**

| CS-g-oligo(NiPAam) copolymer | DLS | NTA | |
|---|---|---|---|
| | $D_h$ (nm) | $D_h$ (nm) | Concentration ($10^8$ Particles/mL) |
| CS-N52 | 137 | 188 | 17 |

### EXAMPLE 11

### Non-covalent corona crosslinking of PVA-g-oligo(NiPAam) micelles

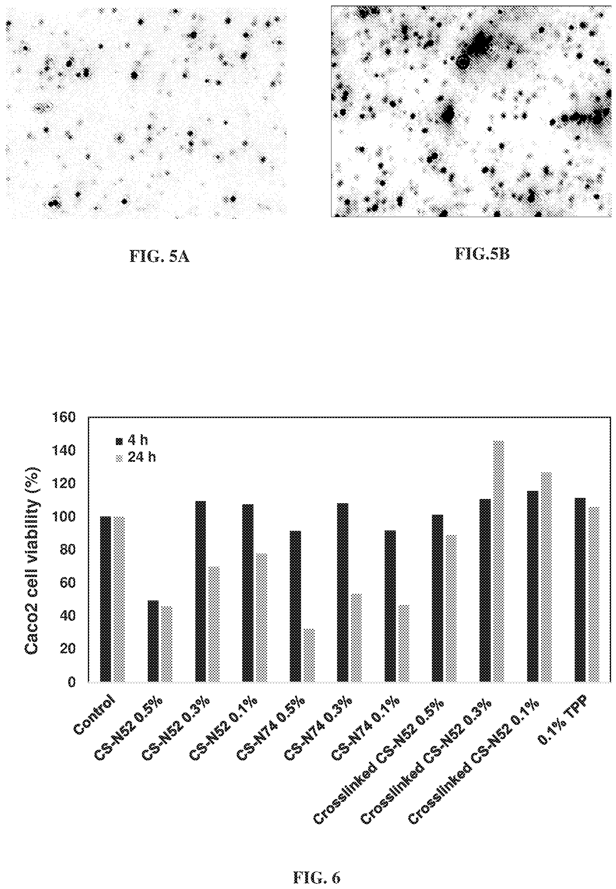

In exemplary procedures, PVA-g-oligo(NiPAam) micelles (0.008% and 0.08%) were stabilized at 37°C for at least 24 h. Then, boric acid (BA, Sigma-Aldrich) solution (0.1% w/v; volumes of 1-5 μL BA solution per mL of polymeric solution) was added at 37°C and the system stored for at least 6 h to allow the complexation crosslinking of the micellar corona. To confirm the crosslinking, polymeric micelles were stored at 25°C for 12 h and visualized by NTA **(Figures 5).**

Table 11 below presents the particle size and concentration of non-covalently crosslinked PVA-N40 polymeric micelles (0.015% w/v) as determined by DLS and NTA, at room temperature.

**Table 11**

| PVA-g-oligo(NiPAam) copolymer | DLS | NTA | |
|---|---|---|---|
| | $D_h$ (nm) | $D_h$ (nm) | Concentration ($10^8$ Particles/mL) |
| PVA-N40 | 267 | 175 | 2.6 |

*EXAMPLE 12*

***In vitro mucoadhesion of non-crosslinked and non-covalently crosslinked CS-g-oligo(NiPAam) micelles***

In exemplary procedures, the mucoadhesion of the non-crosslinked and non-covalently crosslinked CS-g-oligo(NiPAam) micelles was determined *in vitro* using the mucin solution method that is based on the growth of the micellar $D_h$ as a result of the interaction and agglomeration in the presence of soluble mucin. For this, mucin aqueous solution was prepared at room temperature. Then, the solution was centrifuged (1,500 RPM) and filtered with 0.45 $\mu$m filter to render a 0.014 % w/v final mucin concentration and incubated at 37°C for 12 h. Then, fresh non-crosslinked and non-covalently crosslinked CS-N52 polymeric micelles (2 mL, 0.016 % w/v) were added to the mucin solution (2 mL), incubated at 37°C and the $D_h$ followed up by DLS. Then, a mucoadhesion index (MI) was calculated according to the following equation:

$$MI = D_{5h}/D_{0h}$$

Where $D_{0h}$ and $D_{4h}$ are the $D_h$ of particles before (t = 0 h) and after (t = 5 h) the incubation with mucin, respectively.
Table 12 below summarizes MI values of non-crosslinked and non-covalently crosslinked CS-N52 micelles (0.008% w/v) in contact with mucin (0.007% w/v) as determined by DLS, at 37°C. A sharp increase in the size was observed by DLS for both samples.

**Table 12**

| CS-g-oligo(NiPAam) polymeric micelles | MI |
|---|---|
| Non-crosslinked CS-N52 | 5.3 |
| Non-covalently crosslinked CS-N52 | 3.0 |

*EXAMPLE 13*

***In vitro mucoadhesion of non-crosslinked and non-covalently crosslinked PVA-g-oligo(NiPAam) micelles***

In exemplary procedures, the mucoadhesion of the non-crosslinked and non-covalently crosslinked PVA-g-oligo(NiPAam) micelles was determined *in vitro* using the mucin solution method as described in Example 12.
Table 13 below summarizes MI values of PVA-N38 micelles (0.015% w/v) in contact with mucin (0.007% w/v) as determined by DLS, at 37°C. A sharp increase in the size was observed by DLS for both samples.

**Table 13**

| PVA-g-oligo(NiPAam) polymeric micelles | MI |
|---|---|
| Non-crosslinked PVA-N40 | 1.8 |
| Non-covalently crosslinked PVA-N40 | 2.8 |

*EXAMPLE 14*

***In vitro cell compatibility of CS-g-oligo(NiPAam) micelles***

Since these polymeric micelles are envisioned for the administration by mucosal routes, including the oral one, their cell compatibility before and after corona-crosslinking was evaluated in a Caco2 cell line (ATCC® HTB-37™) obtained from American Type Culture Collection (ATCC®, Manassas, VA, USA).
In exemplary embodiments, cells were cultured in Eagle's Minimum Essential Medium (EMEM, Life Technologies Corp., Carlsbad, CA, USA) supplemented with L-glutamine, 10% heat-inactivated fetal bovine serum (FBS, Sigma-Aldrich) and penicillin/streptomycin (5 mL of a commercial mixture of 100 U/mL of penicillin + 100$\mu$g /mL streptomycin per 500 mL medium, Sigma-Aldrich) and maintained at 37°C in a humidified 5% $CO_2$ atmosphere and split 1:10 into fresh media every three-four days. Cells were harvested by trypsinization (trypsin-EDTA 0.25%, Sigma-Aldrich) and the number of live cells was quantified the trypan blue (1%, Sigma-Aldrich) exclusion assay and a Neubauer chamber. To determine the compatibility of the polymeric micelles (expressed as percentage with respect to a blank incubated with culture medium), cells were cultured in 96-well plates ($5 \times 10^3$ cells/well) and allowed to attach over 72 h. The medium in each well was replaced by different samples (concentration between 0.1%, 0.3% and 0.5% in PBS + medium) and cells incubated over 4 and 24 h. Then, the medium was removed and fresh medium (100 $\mu$L) and 25 $\mu$L of sterile 3-(4,5-dimethylthiazol-2-yl)-2,5-diphenyltetrazolium bromide solution (MTT, 5 mg/mL; Sigma-Aldrich) was added. Samples were incubated for 4 h (37°C, 5% $CO_2$), the supernatant was removed and formazan crystals dissolved with dimethyl sulfoxide (DMSO, 200 $\mu$L). Finally, 100 $\mu$L of the DMSO solution was pipetted into a new 96-well plate and the absorbance at 570 nm (reference of 650 nm) was measured with a UV-Visible microplate reader (Multiskan GO, Thermo Scientific, Waltham, MA, USA). The percentage of live cells was estimated with respect to a control treated only with culture medium and that was considered 100% viability.

**Figure 6** summarizes the percentage of viability using and crosslinked and non-crosslinked polymeric micelles with respect to an only-medium control that was considered 100% viability. The highly biocompatible crosslinking (TPP) did not affect the viability of the cells. Moreover, crosslinking increased the compatibility of the micelles.

## EXAMPLE 15

### In vitro cell compatibility of PVA-g-oligo(NiPAam) micelles

In exemplary procedures, the cell compatibility of non-crosslinked PVA-N56 and PVA-N68 micelles was determined *in vitro* using the Caco2 cells method as described in Example 13.

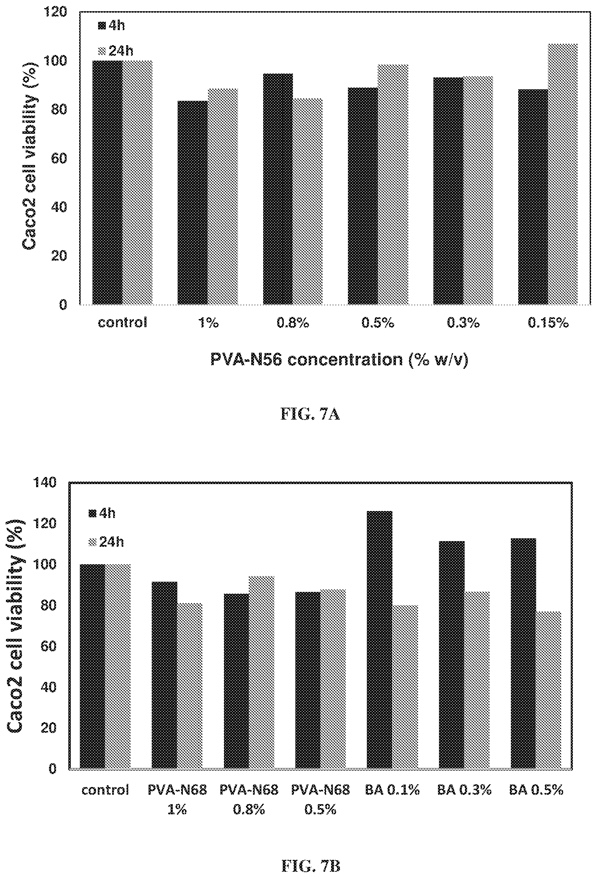

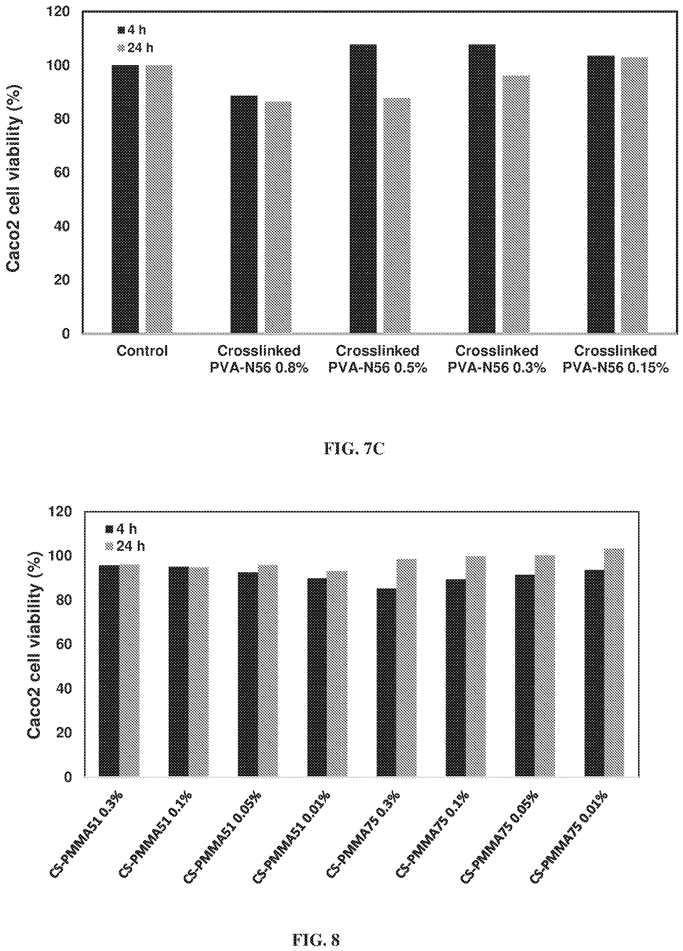

**Figure 7** summarizes the percentage of viability of non-crosslinked **(Figure 7A**, **7B)** and crosslinked **(Figure 7C)** polymeric micelles with respect to an only-medium control that was considered 100% viability. In addition, the cell viability after exposure to BA (the crosslinker) was also assessed **(Figure 7B).**

## EXAMPLE 16

### In vitro cell compatibility of CS-g-PMMA micelles

In exemplary procedures, the cell compatibility of non-crosslinked CS-PMMA micelles was determined *in vitro* using the Caco2 cells method as described in Example 12.

**Figure 8** summarizes the percentage of viability of non-crosslinked polymeric micelles with respect to an only-medium control that was considered 100% viability. The crosslinking with TPP is expected to improve the cell compatibility of the micelles.

## EXAMPLE 17

### Microscopy visualization of CS-g-oligo(NiPAam) micelles

The morphology and the nanostructure of drug-free and drug-loaded non-crosslinked and non-covalently crosslinked polymeric micelles (0.08-1% w/v) was analyzed by high-resolution scanning electron microscopy and cryo-transmission electron microscopy.

(a) *High-resolution scanning electron microscopy (HR-SEM).* Micellar suspensions (0.016% w/v, 15 $\mu$L) were drop-casted on a carbon tape. Then, the drops were air-dried overnight and analyzed in a Zeiss Ultra-Plus FEG 0.02-30 kV SEM (Cambridge, MA, USA).

(b) *Cryo-transmission electron microscopy (cryo-TEM).* Samples (10 $\mu$L) were prepared in the controlled environment vitrification system, equilibrated at 25 °C and examined in a FEI T12 G2 Cryo-TEM at 120 kV. Images were digitally recorded with a Gatan MultiScan 791 camera using the DigitalMicrograph software (Gatan, Inc.) under low dose conditions.

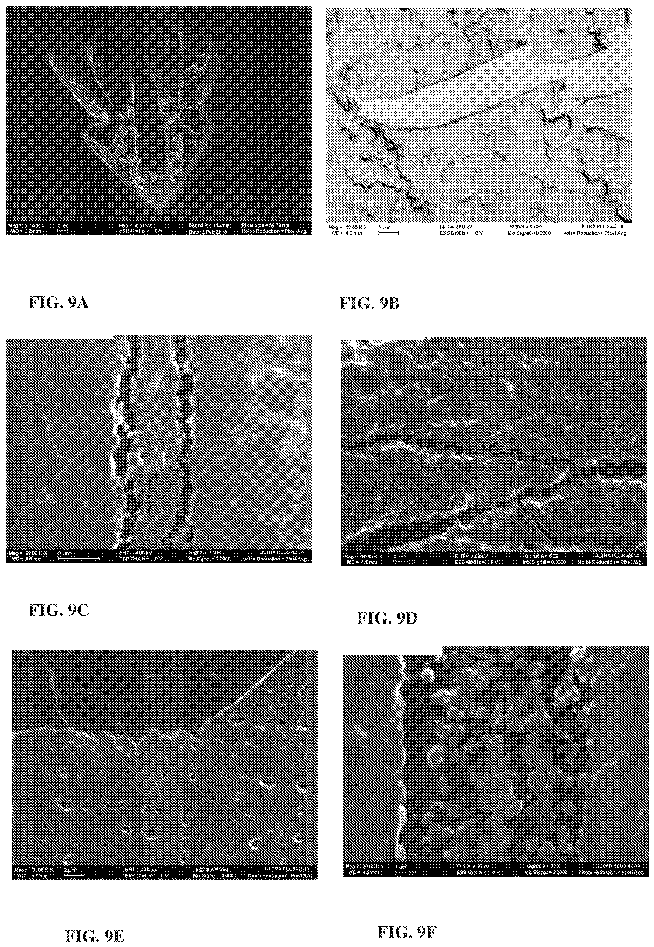

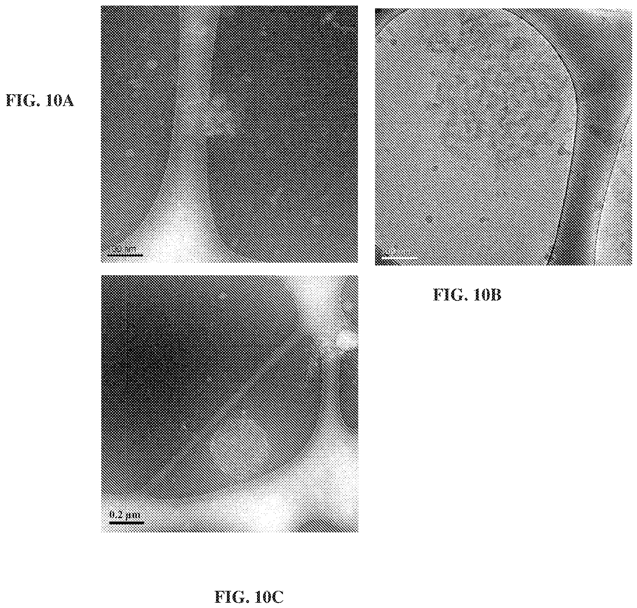

**Figures 9A-F** shows the spherical morphology and the physical stability of CS-N52 micelles after TPP cross-linking with respect to non-crosslinked counterparts, as determined. The stability was also observed by cryo-TEM (**Figure 10A-C**).

## EXAMPLE 18

### Microscopy visualization of PVA-g-oligo(NiPAam) micelles

In exemplary procedures, non-crosslinked PVA-g-oligo(NiPAAm) micelles were visualized by HR-SEM as in Example 15.

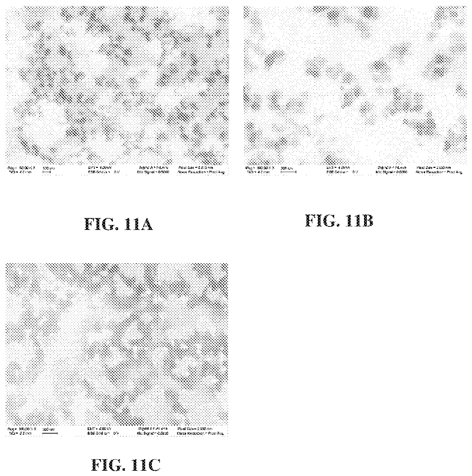

**Figures 11A-C** show the spherical morphology of non-crosslinked PVA-N52, as visualized by HR-SEM.

*EXAMPLE 19*

*Encapsulation of efavirenz in CS-g-oligo(NiPAam) micelles*

The encapsulation capacity of the micelles was studied with the antiretroviral efavirenz. For this, 1%, 3%, 5% and 10% w/v CS-N52 and 1% CS-N74 polymeric micelles were prepared and 2-10 mg of drug per mL of micellar suspension added. The suspension was magnetically stirred at 37°C (48 h), filtered (1.2 μm, Minisart® NML cellulose acetate, Sartorius AG, Goettingen, Germany) to remove insoluble drug, the filtrate diluted (1/10) in distilled water and 10 mL extracted with dichloromethane (3 x 20 mL). The organic solution was evaporated under vacuum, re-dissolved in methanol and the absorbance measured in microplate spectrophotometer (λ = 248 nm). Then, the EFV concentration was calculated by interpolation in a calibration curve in the range of 0.02-80 μg/mL ($R^2$ >0.9999). Finally, the solubility factor ($f_s$) was determined according to

$$f_s = {S_a}/{S_i}$$

where $S_a$ is the apparent solubility of active agent (herein: efavirenz) in the polymeric micelles and $S_i$ is the intrinsic solubility of the active agent (efavirenz) in a polymer-

**Table 14**

| CS-g-oligo(NiPAam) copolymer | Polymer concentration (% w/v) | Non-crosslinked EFV-loaded | | | | Crosslinked EFV-loaded | |
|---|---|---|---|---|---|---|---|
| | | $D_h$ (nm) | PDI | Sa (μg/mL) | $f_s$ | $D_h$ (nm) | PDI |
| **CS-N52** | **1** | 310 | 0.30 | 1140 | 163 | 248 | 0.2 |
| | **3** | 327 | 0.30 | 3777 | 540 | - | - |
| | **5** | 543 | 0.30 | 8127 | 1161 | - | - |
| | **10** | 1038 | 0.40 | 8497 | 1214 | - | - |
| **CS-N74** | **1** | - | - | 2100 | 300 | - | - |

free medium (7 μg/mL), at 37 °C. Measurements were performed in triplicate and the results are expressed as the mean ± S.D. Efavirenz-loaded micelles before and after ionotropic crosslinking were characterized for $D_h$, PDI and Z-potential by DLS (see above).

**Table 14** summarizes the encapsulation data of efavirenz within CS-N52 and CS-N74 polymeric micelles.

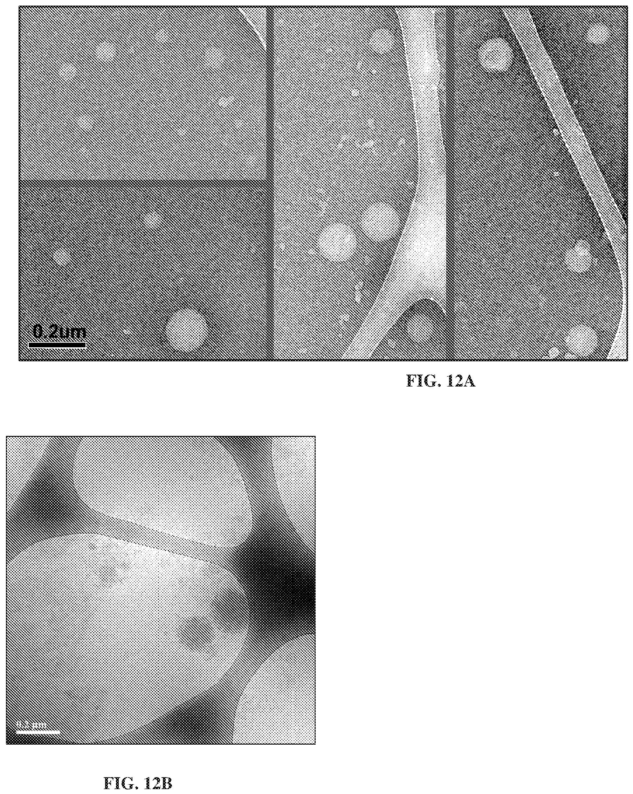

**Figures 12A-B** present the spherical morphology and the physical stability of efavirenz-loaded CS-N52 micelles before **(Figure 12A)** and after TPP crosslinking **(Figure 12B),** as visualized by cryo-TEM.

*EXAMPLE 20*

*In vitro release of efavirenz from CS-g-oligo(NiPAam) micelles*

In exemplary procedures, the drug release profile from the polymeric micelles was evaluated *in vitro* using the dialysis membrane method. For this, a 1% w/v efavirenz-loaded CS-N52 micelles diluted (1:50) in buffer acetate (pH 5) and then poured (50 mL) into dialysis membranes (regenerated cellulose dialysis membranes; MWCO of 12-14 kDa) and immersed into phosphate buffer saline buffer (PBS, pH 7.4, 1500 mL) and incubated at 37 °C under gentle magnetic stirring. Every hour (1-24 h) a specimen was taken from the PBS medium (30 mL) for evaluating the amount of the released drug and replaced with fresh and pre-heated (37 °C) PBS medium (30 mL). Each specimen was then extracted with dichloromethane (3 x 30 mL). The organic solution was evaporated under vacuum, re-dissolved in methanol (1 mL) and the efavirenz concentration was measured as described hereinabove. The percentage of the released drug was determined according to the following equation:

$$\% \, Release \, efavirenz = \frac{m_t + \sum_{n=0}^{n=t-1} m_n}{m_{total}} \times 100 \; ; \; t > 0$$

where $m_t$ is the efavirenz mass in the PBS medium (1500 mL) in time t (1-24 h) and $m_{total}$ is the efavirenz mass in the polymeric micelles. Measurements were performed in triplicate. The release profile from ionotropically-crosslinked polymeric micelles was evaluated as well. The same method for evaluation was used with the following addition.

The 1% w/v efavirenz-loaded CS-N52 micelles were diluted (1:50) in buffer acetate (pH 5), crosslinked with 20 μL/mL TPP solution (0.1% w/v) and incubated for 12h. Then, poured (50 mL) into dialysis membranes following the same steps as introduced for non-crosslinked polymeric micelles.

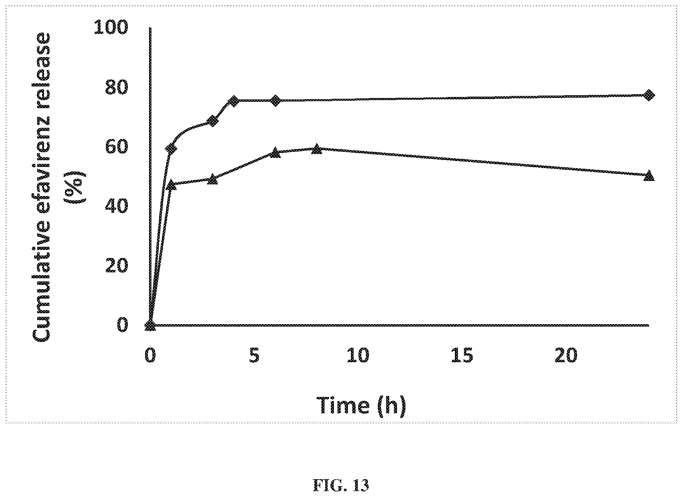

**Figure 13** shows the release profile from both non-crosslinked and ionotropically-crosslinked polymeric micelles.

## Claims

1. A composition-of-matter comprising one or more amphiphilic graft polymers, said amphiphilic graft polymers comprising a hydrophobic domain attached to a side chain group of a hydrophilic domain, wherein:

   (a) said hydrophobic domain is selected from the group consisting of a polyester, a polyacrylamide, a polymethacrylate, and a polyacrylate or a combination thereof;
   (b) said hydrophilic domain is selected from the group consisting of a polycarboxylic acid, a polyol, a polysaccharide or a combination thereof;

   and said one or more amphiphilic graft polymers are in a form of a multi-micellar particle comprising a hydrophobic core and a non-covalently cross-linked hydrophilic corona, wherein said non-covalently cross-linked is via any one of: ionotropic cross-linking, and complexation interactions.

2. The composition-of-matter of claim 1, wherein said core is **characterized by** a structure having a cavity that ranges from 1 nm to 10 μm in diameter, optionally said core being **characterized by** a structure having a cavity that ranges from 50 nm to 300 nm in diameter.

3. The composition-of-matter of any one of claims 1 or 2, wherein said hydrophobic domain comprises a polymer selected from the group consisting of: N-isopropylacrylamide, methyl acrylate, ethyl acrylate, propyl acrylate, butyl acrylate, methyl methacrylate, ethyl methacrylate, propyl methacrylate, butyl methacrylate, acrylic acid, methacrylic acid, quaternary ammonium-modified acrylate, quaternary ammonium modified-methacrylate, acrylamide, caprolactone, lactide, valeronolactone, and optionally wherein the hydrophobic domain is in the range of 10% to 90%, by weight, of the amphiphilic graft polymer.

4. The composition-of-matter of any one of claims 1 to 3, wherein a hydrophobic polymeric backbone of said hydrophobic domain is attached to a side-chain group of said polymeric backbone of said hydrophilic domain *via:* (a) a bifunctional coupling agent selected from the group consisting of: diisocyanates, disilanes, dialkoxysilanes, diglycidyl ethers; (b) a coupling agent comprising a higher functionality than two; or (c) condensation agent selected from the group consisting of dicyclohexylcarbodiimide, O-(benzotriazol-1-yl)-N,N,N',N'-tetramethyluronium tetrafluoroborate, or a combination thereof.

5. The composition-of-matter of any one of claims 1 to 4, wherein said hydrophilic domain comprises one or more monomeric units derived from a polymer selected from the group consisting of: polyvinyl alcohol (PVA), chitosan, alginate, galactomannan, hydroxyproylmethyl cellulose, carboxymethyl cellulose, glucomannan, guar gum, xanthan gum, pectin, hyaluronic acid, chondroitin sulfate, dermatan sulfate.

6. The composition-of-matter of any one of claims 1 to 5, characterized as being adhesive to a mucosal tissue.

7. The composition-of-matter of any one of claims 1 to 6, wherein said amphiphilic graft polymers are characterized as being substantially non-biodegradable in a physiologic environment for a period of at least 24 hours.

8. The composition-of-matter of any one of claims 1 to 7, wherein said amphiphilic graft polymers are **characterized by** a hydrophilic-lipophilic balance (HLB) value that ranges from 1 to 24, optionally wherein a plurality of amphiphilic graft polymers is self-assembled so as to form said multi-micellar particle.

9. The composition-of-matter of any one of claims 1 to 8, comprising one or more active agents, each of said active agents being independently encapsulated within said core, optionally wherein said one or more active agents are stably encapsulated within said core in a physiological environment for at least 24 h, optionally, wherein said one or more active agents are selected from the group consisting of a pharmaceutically active agent, a labeling agent, a diagnostic agent, a prophylactic agent, a surface-modifying agent, a tumor-targeting- ligand or moiety, optionally, wherein said one or more active agents are selected from the group consisting of a pharmaceutically active agent, a labeling agent, a diagnostic agent, a prophylactic agent, a surface-modifying agent, a tumor-targeting- ligand or moiety, and optionally wherein said one or more agents are water-insoluble agent.

10. The composition-of-matter of any one of claims 1 to 9, wherein at least portion of said corona is positively or negatively charged, optionally wherein at least portion of said corona is non-covalently linked to one or more material selected from the group consisting of a cation, an anion, a polycation, a polyanion, a complexation agent.

11. The composition-of-matter of claim 9, for use as a medicament.

12. A pharmaceutical product, comprising the composition-of-matter of claim 9, optionally said product being a pharmaceutically acceptable injectable matrix, optionally said product being formulated for oral or nasal administration.

13. A pharmaceutical product, comprising the composition-of-matter of claim 9, for use in the treatment of a medical condition treatable by said therapeutically active agent, and optionally for use in monitoring or treating a medical condition, said product being packaged in a packaging material and identified in print, in or on said packaging material.

14. A process of preparing an amphiphilic graft polymer comprising the steps of:

grafting a hydrophobic polymeric backbone to a hydrophilic polymeric backbone in a dispersion, thereby forming said amphiphilic graft polymer configured to form a hydrophobic core and a hydrophilic corona;
dispersing the amphiphilic graft polymer in an aqueous medium at a concentration above a predefined minimal concentration;
adding an active agent to the dispersion, thereby encapsulating the active agent within the hydrophobic core; and
adding a non-covalent crosslinker to the dispersion, so as to form a non-covalent cross-link of at least portion of the hydrophilic corona, wherein said non-covalent cross-link is via any one of: ionotropic cross-linking, and complexation interactions;
thereby forming said amphiphilic graft polymer being in form of a closed core-corona and self-assembled structure further encapsulating one or more active agents.

15. The process of claim 14, wherein said predefined concentration is critical micelle concentration (CMC), optionally said method further comprising a step of heating an aqueous solution containing said amphiphilic graft polymer to a temperature that ranges from about 30 °C to about 50 °C, prior to the step of adding the active agent to the dispersion, optionally said method further comprising a step of cooling the dispersion to a temperature lower than 30 °C, thereby stabilizing the amphiphilic graft polymer, and optionally said method further comprising a step of diluting the dispersion to a final concentration below the predefined concentration, following the step of adding an active agent to the dispersion thereby stabilizing the amphiphilic graft polymer; further optionally wherein said closed core-corona and self-assembled structure is the multi-micellar particle of any one of claims 1 to 10.

**Patentansprüche**

1. Stoffzusammensetzung, umfassend ein oder mehrere amphiphile Pfropfpolymere, wobei die amphiphilen Pfropfpolymere eine hydrophobe Domäne umfassen, die an eine Seitenkettengruppe einer hydrophilen Domäne gebunden ist, wobei:

(a) die hydrophobe Domäne aus der Gruppe ausgewählt ist, die aus einem Polyester, einem Polyacrylamid, einem Polymethacrylat und einem Polyacrylat oder einer Kombination davon besteht;
(b) die hydrophile Domäne aus der Gruppe ausgewählt ist, die aus einer Polycarbonsäure, einem Polyol, einem Polysaccharid oder einer Kombination davon besteht;

und die ein oder mehreren amphiphilen Pfropfpolymere in Form eines Multimizellen-Teilchens vorliegen, das einen hydrophoben Kern und eine nicht-kovalent vernetzte hydrophile Corona umfasst, wobei die nicht-kovalente Vernetzung über ionotrope Vernetzung oder Komplexierungswechselwirkungen erfolgt ist.

2. Stoffzusammensetzung nach Anspruch 1, bei der der Kern durch eine Struktur mit einem Hohlraum mit einem Durchmesser im Bereich von 1 nm bis 10 $\mu$m gekennzeichnet ist, optional bei der der Kern durch eine Struktur mit einem Hohlraum mit einem Durchmesser im Bereich von 50 nm bis 300 nm gekennzeichnet ist.

3. Stoffzusammensetzung nach Anspruch 1 oder 2, bei der die hydrophobe Domäne ein Polymer umfasst, das aus der Gruppe ausgewählt ist, die aus N-Isopropylacrylamid, Methylacrylat, Ethylacrylat, Propylacrylat, Butylacrylat, Methylmethacrylat, Ethylmethacrylat, Propylmethacrylat, Butylmethacrylat, Acrylsäure, Methacrylsäure, mit quaternärem Ammonium modifiziertem Acrylat, mit quaternärem Ammonium modifiziertem Methacrylat, Acrylamid, Caprolacton, Lactid, Valeronolacton besteht, und optional, bei der die hydrophobe Domäne im Bereich von 10 bis 90 Gew.-% des amphiphilen Pfropfpolymers liegt.

4. Stoffzusammensetzung nach einem der Ansprüche 1 bis 3, bei der ein hydrophobes Polymergerüst der hydrophoben Domäne an eine Seitenkettengruppe des Polymergerüsts der hydrophilen Domäne gebunden ist über: (a) ein bifunktionelles Kopplungsmittel, ausgewählt aus der Gruppe, die aus Diisocyanaten, Disilanen, Dialkoxysilanen, Diglycidylethern besteht; (b) ein Kopplungsmittel mit einer Funktionalität von mehr als zwei; oder (c) ein Kondensationsmittel, ausgewählt aus der Gruppe, die aus Dicyclohexylcarbodiimid, O-(Benzotriazol-1-yl)-N,N,N',N'-tetramethyluroni-umtetrafluoroborat oder einer Kombination davon besteht.

5. Stoffzusammensetzung nach einem der Ansprüche 1 bis 4, bei der die hydrophile Domäne eine oder mehrere Monomereinheiten umfasst, die von einem Polymer abgeleitet sind, das aus der Gruppe ausgewählt ist, die aus Polyvinylalkohol (PVA), Chitosan, Alginat, Galactomannan, Hydroxypropylmethylcellulose, Carboxymethylcellulose, Glucomannan, Guargummi, Xanthangummi, Pektin, Hyaluronsäure, Chondroitinsulfat, Dermatansulfat besteht.

6. Stoffzusammensetzung nach einem der Ansprüche 1 bis 5, **dadurch gekennzeichnet, dass** sie an einem Schleimhautgewebe haftet.

7. Stoffzusammensetzung nach einem der Ansprüche 1 bis 6, bei der die amphiphilen Pfropfpolymere **dadurch gekennzeichnet sind, dass** sie in einer physiologischen Umgebung über einen Zeitraum von mindestens 24 Stunden im Wesentlichen nicht biologisch abbaubar sind.

8. Stoffzusammensetzung nach einem der Ansprüche 1 bis 7, bei der die amphiphilen Pfropfpolymere durch einen HLB-Wert (Hydrophilic-Lipophilic-Balance) im Bereich von 1 bis 24 gekennzeichnet sind, optional bei der eine Mehrzahl von amphiphilen Pfropfpolymeren selbstorganisiert ist, um das Multimizellen-Teilchen zu bilden.

9. Stoffzusammensetzung nach einem der Ansprüche 1 bis 8, umfassend einen oder mehrere Wirkstoffe, wobei jeder der Wirkstoffe unabhängig in dem Kern verkapselt ist, optional wobei die ein oder mehreren Wirkstoffe in dem Kern in einer physiologischen Umgebung für mindestens 24 h stabil verkapselt sind, optional wobei die ein oder mehreren Wirkstoffe aus der Gruppe ausgewählt sind, die aus einem pharmazeutisch aktiven Wirkstoff, einem Markierungsmittel, einem Diagnosemittel, einem Prophylaxemittel, einem oberflächenmodifizierenden Mittel, einem Tumor-Targeting-Liganden oder einer Tumor-Targeting-Einheit besteht, und optional, wobei die ein oder mehreren Wirkstoffe wasserunlöslich sind.

10. Stoffzusammensetzung nach einem der Ansprüche 1 bis 9, bei der mindestens ein Teil der Corona positiv oder negativ geladen ist, optional bei der mindestens ein Teil der Corona nicht-kovalent an ein oder mehrere Materialien gebunden ist, die aus der Gruppe ausgewählt sind, die aus einem Kation, einem Anion, einem Polykation, einem Polyanion und einem Komplexbildner besteht.

11. Stoffzusammensetzung nach Anspruch 9 zur Verwendung als Medikament.

12. Pharmazeutisches Produkt, umfassend die Stoffzusammensetzung nach Anspruch 9, optional wobei das Produkt eine pharmazeutisch verträgliche injizierbare Matrix ist, optional wobei das Produkt für eine orale oder nasale Verabreichung formuliert ist.

13. Pharmazeutisches Produkt, umfassend die Stoffzusammensetzung nach Anspruch 9, zur Verwendung bei der Behandlung eines medizinischen Zustands, der durch den therapeutischen Wirkstoff behandelbar ist, und optional zur Verwendung bei der Überwachung oder Behandlung eines medizinischen Zustands, wobei das Produkt in einem Verpackungsmaterial verpackt ist und in oder auf dem Verpackungsmaterial in gedruckter Form identifiziert ist.

14. Verfahren zur Herstellung eines amphiphilen Pfropfpolymers, umfassend die Schritte:

Pfropfen eines hydrophoben polymeren Grundgerüsts auf ein hydrophiles polymeres Grundgerüst in einer Dispersion, wodurch das amphiphile Pfropfpolymer gebildet wird, das konfiguriert ist, um einen hydrophoben Kern und eine hydrophile Corona zu bilden;

Dispergieren des amphiphilen Pfropfpolymers in einem wässrigen Medium bei einer Konzentration über einer vordefinierten Mindestkonzentration;

Zugeben eines Wirkstoffs zu der Dispersion, wodurch der Wirkstoff in dem hydrophoben Kern eingekapselt wird; und

Hinzufügen eines nicht-kovalenten Vernetzungsmittels zu der Dispersion, um eine nicht-kovalente Vernetzung von mindestens einem Teil der hydrophilen Corona zu bilden, wobei die nicht-kovalente Vernetzung über ionotrope Vernetzung oder Komplexierungswechselwirkungen erfolgt;

wodurch das amphiphile Pfropfpolymer in Form einer Corona mit geschlossenem Kern und einer selbstorganisierten Struktur gebildet wird, die ferner einen oder mehrere Wirkstoffe einkapselt.

15. Verfahren nach Anspruch 14, wobei die vordefinierte Konzentration die kritische Mizellenkonzentration (CMC) ist, wobei optional das Verfahren vor dem Schritt des Hinzufügens des Wirkstoffs zu der Dispersion ferner einen Schritt des Erhitzens einer wässrigen Lösung, die das amphiphile Pfropfpolymer enthält, auf eine Temperatur im Bereich von etwa 30 °C bis etwa 50 °C umfasst, wobei optional das Verfahren ferner einen Schritt des Kühlens der Dispersion auf eine Temperatur unter 30 °C umfasst, wodurch das amphiphile Pfropfpolymer stabilisiert wird, und optional das Verfahren ferner im Anschluss an den Schritt des Hinzufügens eines Wirkstoffs zu der Dispersion einen Schritt des Verdünnens der Dispersion auf eine Endkonzentration unterhalb der vordefinierten Konzentration umfasst, wodurch das amphiphile Pfropfpolymer stabilisiert wird; ferner optional, bei dem die Corona mit geschlossenem Kern und selbstorganisierte Struktur das Multimicellen-Teilchen nach einem der Ansprüche 1 bis 10 ist.

## Revendications

1. Composition de matière comprenant un ou plusieurs polymères greffés amphiphiles, lesdits polymères greffés amphiphiles comprenant un domaine hydrophobe attaché à un groupe à chaîne latérale d'un domaine hydrophile, dans laquelle :

(a) ledit domaine hydrophobe est choisi dans le groupe constitué d'un polyester, d'un polyacrylamide, d'un polyméthacrylate et d'un polyacrylate ou d'une combinaison de ceux-ci ;
(b) ledit domaine hydrophile est choisi dans le groupe constitué d'un acide polycarboxylique, d'un polyol, d'un polysaccharide ou d'une combinaison de ceux-ci ;

et lesdits un ou plusieurs polymères greffés amphiphiles se présentent sous la forme d'une particule multi-micellaire comprenant un noyau hydrophobe et une couronne hydrophile réticulée non covalente, dans laquelle ladite réticulation non covalente est réalisée par l'une quelconque d'une réticulation ionotrope et d'interactions de complexation.

2. Composition de matière selon la revendication 1, dans laquelle ledit noyau est **caractérisé par** une structure comportant une cavité présentant un diamètre dans une plage de 1 nm à 10 $\mu$m, facultativement ledit noyau étant **caractérisé par** une structure comportant une cavité présentant un diamètre dans une plage de 50 nm à 300 nm.

3. Composition de matière selon la revendication 1 ou 2, dans laquelle ledit domaine hydrophobe comprend un polymère choisi dans le groupe constitué de N-isopropylacrylamide, d'acrylate de méthyle, d'acrylate d'éthyle, d'acrylate de propyle, d'acrylate de butyle, de méthacrylate de méthyle, de méthacrylate d'éthyle, de méthacrylate de propyle, de méthacrylate de butyle, d'acide acrylique, d'acide méthacrylique, d'acrylate modifié par ammonium quaternaire, de méthacrylate modifié par ammonium quaternaire, d'acrylamide, caprolactone, de lactide, valérolactone, et facultativement dans laquelle le domaine hydrophobe est dans la plage de 10 % à 90 %, en poids, du polymère greffé amphiphile.

4. Composition de matière selon l'une quelconque des revendications 1 à 3, dans laquelle un squelette polymère hydrophobe dudit domaine hydrophobe est attaché à un groupe à chaîne latérale dudit squelette polymère dudit domaine hydrophile via : (a) un agent de couplage bifonctionnel choisi dans le groupe constitué des diisocyanates, disilanes, dialkoxysilanes, éthers diglycidyliques ; (b) un agent de couplage comprenant une fonctionnalité supérieure à deux ; ou (c) un agent de condensation choisi dans le groupe constitué de dicyclohexylcarbodiimide, de tétrafluoroborate de O-(benzotriazol-1-yl)-N,N,N',N'-tétraméthyluronium, ou d'une combinaison de ceux-ci.

**5.** Composition de matière selon l'une quelconque des revendications 1 à 4, dans laquelle ledit domaine hydrophile comprend une ou plusieurs unités monomères dérivées d'un polymère choisi dans le groupe constitué de l'alcool polyvinylique (PVA), de chitosane, d'alginate, de galactomannane, de cellulose de hydroxypropylméthyle, de cellulose de carboxyméthyle, de glucomannane, de gomme de guar, de gomme de xanthane, de pectine, d'acide hyaluronique, de sulfate de chondroïtine, et de sulfate de dermatane.

**6.** Composition de matière selon l'une quelconque des revendications 1 à 5, **caractérisée en ce qu'**elle est adhésive à un tissu muqueux.

**7.** Composition de matière selon l'une quelconque des revendications 1 à 6, dans laquelle lesdits polymères greffés amphiphiles sont **caractérisés en ce qu'**ils sont sensiblement non biodégradables dans un environnement physiologique pendant une période d'au moins 24 heures.

**8.** Composition de matière selon l'une quelconque des revendications 1 à 7, dans laquelle lesdits polymères greffés amphiphiles sont **caractérisés par** une valeur de rapport hydrophile-lipophile (HLB) dans une plage de 1 à 24, facultativement dans laquelle une pluralité de polymères greffés amphiphiles sont auto-assemblés de manière à former ladite particule multi-micellaire.

**9.** Composition de matière selon l'une quelconque des revendications 1 à 8, comprenant un ou plusieurs agents actifs, chacun desdits agents actifs étant encapsulé indépendamment à l'intérieur dudit noyau, facultativement dans laquelle lesdits un ou plusieurs agents actifs sont encapsulés de manière stable à l'intérieur dudit noyau dans un environnement physiologique pendant au moins 24 heures, facultativement dans laquelle lesdits un ou plusieurs agents actifs sont choisis dans le groupe constitué d'un agent actif pharmaceutiquement, d'un agent de marquage, d'un agent de diagnostic, d'un agent prophylactique, d'un agent de modification de surface, d'un ligand ou fragment ciblant une tumeur, facultativement dans laquelle lesdits un ou plusieurs agents actifs sont choisis dans le groupe constitué d'un agent actif pharmaceutiquement, d'un agent de marquage, d'un agent de diagnostic, d'un agent prophylactique, d'un agent de modification de surface, d'un ligand ou fragment ciblant une tumeur, et facultativement dans laquelle lesdits un ou plusieurs agents actifs sont des agents insolubles dans l'eau.

**10.** Composition de matière selon l'une quelconque des revendications 1 à 9, dans laquelle au moins une partie de ladite couronne est de charge positive ou négative, facultativement dans laquelle au moins une partie de ladite couronne est liée de manière non covalente à un ou plusieurs matériaux choisis dans le groupe constitué d'un cation, d'un anion, d'un polycation, d'un polyanion, d'un agent de complexation.

**11.** Composition de matière selon la revendication 9, pour son utilisation comme médicament.

**12.** Produit pharmaceutique, comprenant la composition de matière selon la revendication 9, facultativement ledit produit étant une matrice injectable acceptable pharmaceutiquement, facultativement ledit produit étant formulé pour une administration orale ou nasale.

**13.** Produit pharmaceutique, comprenant la composition de matière selon la revendication 9, pour son utilisation dans le traitement d'un problème de santé traitable par ledit agent actif thérapeutiquement, et facultativement pour son utilisation dans la surveillance ou le traitement d'un problème de santé, ledit produit étant conditionné dans un matériau de conditionnement et identifié par impression, dans ou sur ledit matériau de conditionnement.

**14.** Procédé de préparation d'un polymère greffé amphiphile comprenant les étapes suivantes :

la greffe d'un squelette polymère hydrophobe sur un squelette polymère hydrophile dans une dispersion, en formant ainsi ledit polymère greffé amphiphile configuré pour former un noyau hydrophobe et une couronne hydrophile ;
la dispersion du polymère greffé amphiphile dans un milieu aqueux à une concentration supérieure à une concentration minimale prédéfinie ;
l'ajout d'un agent actif à la dispersion, en encapsulant ainsi l'agent actif à l'intérieur du noyau hydrophobe ; et
l'ajout d'un agent réticulant non covalent à la dispersion, de manière à former une réticulation non covalente d'au moins une partie de la couronne hydrophile, dans lequel ladite réticulation non covalente est réalisée par l'une quelconque d'une réticulation ionotrope et d'interactions de complexation ;
la formation ainsi dudit polymère greffé amphiphile sous la forme d'une structure auto-assemblée de noyau-couronne fermée encapsulant en outre un ou plusieurs agents actifs.

**15.** Procédé selon la revendication 14, dans lequel ladite concentration prédéfinie est une concentration micellaire critique (CMC), facultativement ledit procédé comprenant en outre une étape de chauffage d'une solution aqueuse contenant ledit polymère greffé amphiphile à une température dans une plage d'environ 30 °C à environ 50 °C avant l'étape d'ajout de l'agent actif à la dispersion, facultativement ledit procédé comprenant en outre une étape de refroidissement de la dispersion à une température inférieure à 30 °C en stabilisant ainsi le polymère greffé amphiphile, et facultativement ledit procédé comprenant en outre une étape de dilution de la dispersion à une concentration finale inférieure à la concentration prédéfinie, suivant l'étape d'ajout d'un agent actif à la dispersion en stabilisant ainsi le polymère greffé amphiphile ; facultativement en outre dans laquelle ladite structure auto-assemblée noyau-couronne fermée est la particule multi-micellaire selon l'une quelconque des revendications 1 à 10.

FIG. 1A

FIG. 1B

$$y = -7.9414x + 1.0655$$
$$R^2 = 0.982$$

FIG. 1C

$T_g = 142°C$    CS-N62

$T_g = 139°C$    CS-N52

Pure CS

FIG. 1D

FIG. 2A

FIG. 2B

FIG. 3A

FIG. 3B

FIG. 4A

FIG. 4B

FIG. 5A

FIG.5B

FIG. 6

FIG. 7A

FIG. 7B

FIG. 7C

FIG. 8

FIG. 9A

FIG. 9B

FIG. 9C

FIG. 9D

FIG. 9E

FIG. 9F

FIG. 10A

FIG. 10B

FIG. 10C

FIG. 11A

FIG. 11B

FIG. 11C

FIG. 12A

FIG. 12B

FIG. 13

**EP 3 313 374 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5173322 A
- WO 2003105607 A
- WO 2001087227 A
- US 2007253899 A

### Non-patent literature cited in the description

- **TORCHILIN V.P. et al.** *Wires Nanomed Nanobiotechnol.*, 2015, vol. 7, 691-707
- **SOSNIK A. et al.** *Biotechnology Advances, Mrine Drugs*, 2015, vol. 35, 1380-1392
- **SZYMANSKA E. et al.** *Mar. Drugs*, 2015, vol. 13, 1819-18446
- **G. ODIAN**. Principles of Polymerization. John Wiley & Sons, 1991, 108
- Remington's Pharmaceutical Sciences. Mack Publishing Co.
- **FINGL et al.** *The Pharmacological Basis of Therapeutics*, 1975, 1